(19) Europäisches Patentamt / European Patent Office / Office européen des brevets

(11) **EP 3 418 391 B1**

(12) **EUROPEAN PATENT SPECIFICATION**

(45) Date of publication and mention of the grant of the patent:
**28.09.2022 Bulletin 2022/39**

(21) Application number: **17382394.9**

(22) Date of filing: **23.06.2017**

(51) International Patent Classification (IPC):
*C12Q 1/6804* (2018.01)    *G01N 33/50* (2006.01)
*G01N 33/48* (2006.01)    *C12Q 1/6825* (2018.01)
*G01N 21/64* (2006.01)    *G01N 21/27* (2006.01)
*G16B 25/20* (2019.01)

(52) Cooperative Patent Classification (CPC):
(C-Sets available)
**C12Q 1/6825; C12Q 1/6804; G01N 21/278;
G01N 21/6458; G16B 25/20**    (Cont.)

(54) **METHOD FOR QUANTIFYING PROTEIN COPY-NUMBER**

VERFAHREN ZUR QUANTIFIZIERUNG DER PROTEINVERVIELFÄLTIGUNGSZAHL

PROCÉDÉ DE QUANTIFICATION DE NOMBRE DE COPIES DE PROTÉINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.12.2018 Bulletin 2018/52**

(73) Proprietors:
• **Fundació Institut de Ciències Fotòniques
08860 Castelldefels (Barcelona) (ES)**
• **The Trustees of the Smith College
Northampton, MA 01063 (US)**
• **Fundació Universitaria Balmes
08500 Vic, Barcelona (ES)**

(72) Inventors:
• **LAKADAMYALI, Melike
08860 Castelldefels - Barcelona (ES)**
• **CELLA-ZANACHI, Francesca
08860 Castelldefels - Barcelona (ES)**
• **MANZO, Carlo
08500 Vic - Barcelona (ES)**
• **DERR, Nathan
Northampton, MA 01063 (US)**

(74) Representative: **ABG Intellectual Property Law,
S.L.
Avenida de Burgos, 16D
Edificio Euromor
28036 Madrid (ES)**

(56) References cited:
WO-A1-2017/027818    US-A1- 2013 261 019
US-A1- 2014 057 805

• **CELLA ZANACCHI FRANCESCA ET AL:
"Versatile Super-Resolution Calibration
Standard for Quantifying Protein Copy Number",
BIOPHYSICAL JOURNAL, vol. 112, no. 3, 12
February 2017 (2017-02-12), page 142a,
XP029908564, ISSN: 0006-3495, DOI:
10.1016/J.BPJ.2016.11.782**
• **RALF JUNGMANN ET AL: "Multiplexed 3D
cellular super-resolution imaging with
DNA-PAINT and Exchange-PAINT", NATURE
METHODS, vol. 11, no. 3, 1 March 2014
(2014-03-01), pages 313-318, XP055312024, ISSN:
1548-7091, DOI: 10.1038/nmeth.2835**
• **MINGJIE DAI ET AL: "Optical imaging of
individual biomolecules in densely packed
clusters", NATURE NANOTECHNOLOGY, vol. 11,
no. 9, September 2016 (2016-09), XP002774577,
NATURE PUBLISHING GROUP UK ISSN:
1748-3387, DOI: 10.1038/nnano.2016.95**
• **N. D. DERR ET AL: "Tug-of-War in Motor Protein
Ensembles Revealed with a Programmable DNA
Origami Scaffold", SCIENCE, vol. 338, no. 6107,
2 November 2012 (2012-11-02), pages 662-665,
XP055414483, ISSN: 0036-8075, DOI:
10.1126/science.1226734**

EP 3 418 391 B1

- **SCHUEDER F ET AL: "Universal Super-Resolution Multiplexing by DNA Exchange", ANGEWANDTE CHEMIE - INTERNATIONAL EDITION, vol. 56, no. 14, 3 March 2017 (2017-03-03) , pages 4052-4055, XP002774578, WILEY-VCH VERLAG DEU DOI: 10.1002/ANIE.201611729**
- **ZANACCHI FRANCESCA CELLA ET AL: "A DNA origami platform for quantifying protein copy number in super-resolution", NATURE METHODS, vol. 14, no. 8, August 2017 (2017-08), XP002774579, DOI: 10.1038/nmeth.4342**

(52) Cooperative Patent Classification (CPC): (Cont.)

C-Sets
**C12Q 1/6804, C12Q 2563/107, C12Q 2565/102, C12Q 2565/601;
C12Q 1/6825, C12Q 2563/107, C12Q 2565/102, C12Q 2565/601**

**Description**

**Field of the Invention**

The present invention relates to the field of determining the copy number of proteins in sample imaged with super resolution microscopy.

**Background of the Invention**

Single molecule localization microscopy has become an important tool for imaging intracellular structures and protein complexes with nanoscale spatial resolution (Oddone, A. et al., 2014). Recently, an immense effort has been dedicated to the quantification of super-resolution images (Durisic, N. et al., 2014, Deschout, H.et al., 2014). Among the different quantitative parameters that can be extracted, protein copy-number and stoichiometry have been of particular interest. Single-molecule-based super-resolution methods are uniquely positioned to determine protein copy-numbers, since the single molecule information can be exploited for counting. However, the exact quantification is ultimately impaired by the stochasticity of the labeling method and the complex photophysics of the fluorescent probes. Therefore, it is not surprising that a substantial effort has been dedicated to developing analytical approaches and calibration standards aimed to overcome this challenge. For example, the photophysics of photoactivatable and photoconvertible fluorescent proteins (FPs) have been extensively studied and nano-templates have been developed to calibrate the signal and count FP-tagged proteins (Fricke, F. et al., 2015). Since FPs provide a one to one labeling stoichiometry and have limited blinking or reactivation probability, they are desirable for quantitative imaging. However, a major limitation is imposed by their low photon budget, leading to images with a lower spatial resolution compared to small organic fluorophores, which are the probe of choice for a large number of super-resolution studies. Targeting these bright fluorophores to the protein of interest typically requires immunofluorescent labeling by primary and secondary antibodies. In this case, unfortunately, both the antibody labelling efficiency as well as the number of fluorophores conjugated to the primary or to the secondary antibody are highly stochastic. In addition, fluorophores might undergo repeated blinking or reactivation events. Combined together, these issues pose major challenges for protein-copy quantification. Partial solutions to these challenges have been reported. For example, the fluorophore photophysics can be modelled (Hummer, G. et al.,2016, Rollins, G.C. et al., 2015)) or characterized using single fluorophores conjugated to antibodies or images of sparse spots on the sample (Ricci, M. A., et al., 2015, Ehmann, N. *et al* 2014). In the case of DNA-PAINT approaches - that rely on "on-off" binding of fluorophore-labeled small oligos - the binding kinetics can be modeled and accounted for in the quantification (Jungmann, R. *et al.2016).* Nonetheless, in all cases the unknown stoichiometry of antibody-based labeling, resulting from the stochasticity of fluorophore-antibody and antibody-target binding, largely affects the precision of the final quantification. Therefore, there is an urgent need for versatile calibration standards that take into account not only the fluorophore photophysics but also the antibody and fluorophore labeling stoichiometry. Although in other works, ad hoc calibration standards have allowed quantifying complex structures such as nucleosomes (Ricci, M. A. et al., 2015) there is lack of a general approach toward this problem.

Zanacchi et al. (Biophysical Journal, 2017, vol. 112, no. 3: mentions the development of a new calibration method for quantifying protein copy number with nanoscale resolution comprising the combination of DNA origami and immunofluorescence super-resolution (SR) microscopy.

Jungmann et al, (Nature Methods, 2014, 11: 313-318) describes DNA-paint and Exchange-PAINT technologies.

Dai et al (Nature Nanotechnology, 2016, 11: 798-807() mentions that visualization of single-molecules is achieved and that in the future it will expected the development of the technology for eventually allow quantitative molecular features to be studied. A similar disclosure is shown in US2013/261019.

US 2014/057805 describes a system for calibrating a SR microscope comprising a first DNA origami structure fixed on a support with no labels and a second labelled-DNA origami fixed on a support.

The development of methods able to access a precise molecular counting of protein copy numbers is essential, clearing the way to address several biological questions using super-resolution techniques based on single molecule localization.

**Summary of the Invention**

The invention has been defined in the appended claims.

In a first aspect, the invention relates to a method for obtaining a calibration curve for quantifying protein copy number in fluorescence-based super resolution microscopy which comprises

    a) incubating a DNA origami immobilized on a support, wherein the DNA origami comprises handle oligonucleotides protruding from said DNA origami, said handle oligonucleotides being attached to the DNA origami at predetermined positions and at least one tag, with a protein of interest functionalized with oligonucleotides complementary to the handles protruding from said DNA origami, in conditions allowing the hybridization between the oligonucleotides attached to the DNA origami and the oligonucleotides attached to the protein of interest,

b) recording a super resolution image of the protein of interest which colocalizes with the tag of the DNA origami,

c) clustering the image obtained in step b) and identifying the clusters separated by the distance between the handles to obtain the number clusters in said image obtained in step b),

d) fitting a generic probability distribution function depending on a set of parameters $\mu$ to the distribution of the number of localizations $x$ for one predetermined cluster,

$$f_1(\mu;\ x)$$

and extending it iteratively to larger clusters by using the equation for n= 2, 3...N$_{max}$

$$f_n = f_{n-1} \otimes f_1$$

where $\otimes$ represent the convolution in respect to the x variables between two functions and N$_{max}$ is a predetermined maximum number of clusters, and

e) obtaining a calibration curve by the parameters determined through the fitting procedure described in d), wherein steps d) and e) are executed by a computer and wherein the super resolution image of the protein of interest is obtained by detecting said protein with a fluorescent antibody, fluorescent nanobody or fluorescent halo/snap tag specific for the protein of interest.

In a second aspect, the invention relates to a method for quantifying protein copy number in a sample imaged with super resolution microscopy which comprises, obtaining a statistical parameter of the number of localizations in a sample having the protein of interest and comparing it with the calibration curve obtained for said protein of interest according to the method of the invention.

In a third aspect, the invention relates to a method for determining the percentage of oligomeric state of a protein in a sample imaged with super-resolution microscopy which comprises fitting the overall distribution of the number of localizations obtained in the sample to,

$$g(x) = \sum_{n=1}^{N_{max}} \alpha_n f_n(\mu; x)$$

where $\alpha_n$ represents the weight of the distribution of *n*-mers being $\sum_{n=1}^{N_{max}} \alpha_n = 1$ and f$_n$ is a linear combination of calibration distributions obtained as convoluting function f$_1$, n-times according to:

$$f_n = f_{n-1} \otimes f_1$$

obtained for said protein of interest according to the method of the invention, wherein fittings are performed by optimization of an objective function.

In a fourth aspect, the invention relates to a computer readable storage medium comprising a program stored thereon and instructions which, when the program is executed by a computer, causes the computer to carry out steps d)-e) of the methods of the invention.

In a fifth aspect, the invention relates to a kit comprising

a) a DNA origami attachable to a support comprising handle sequences protruding from said DNA origami and at least one tag, optionally the DNA origami is protected from degradation,

b) reagents suitable for obtaining a super resolution image of a protein of interest, wherein the reagents for obtaining a super resolution image of a protein of interest comprises

i) an antibody or nanobody specific for the protein of interest having at least one fluorophore, or

ii) a primary antibody specific for the protein of interest and a secondary antibody having at least one fluorophore and

c) a computer-readable medium comprising instructions which, when executed by a computer, cause the computer to carry out steps d)-e) of the methods of the

invention.

In a sixth aspect, the invention relates to the use of a kit of the invention for obtaining a calibration curve for quantifying protein copy number in immunofluorescence-based super resolution microscopy, for quantifying protein copy number in a sample imaged with super resolution microscopy and for determining the percentage of oligomeric state of a protein in a sample imaged with super-resolution microscopy.

**Brief description of the Figures**

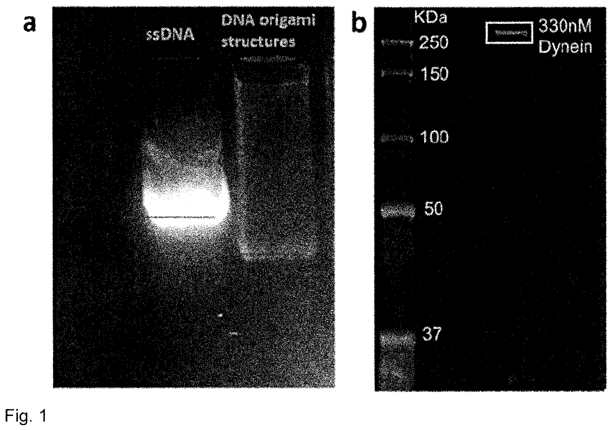

Figure 1: agarose gel electrophoresis of DNA origami and purified Dynein: (a) Folding of the 12 helix chassis was assessed by 2% agarose gel electrophoresis showing the difference between the folded chassis (second lane) compared to the initial scaffold used for the reaction (first lane). (b) Dynein from *Saccharomyces cerevisiae* was purified as previously described (Oddone, A. et al., 2014) obtaining a final concentration of 330nM (white box).

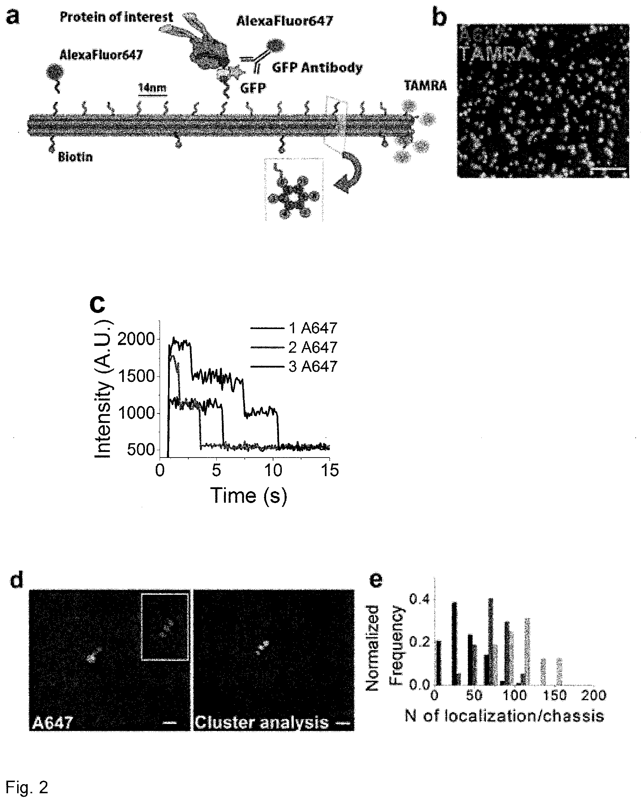

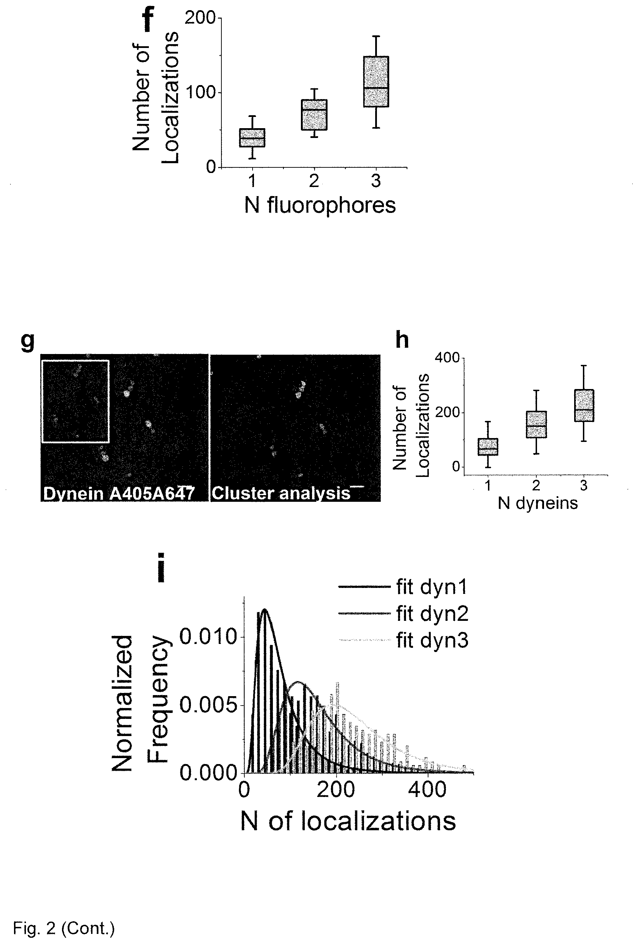

Figure 2. DNA origami calibration: (a) Schematic representation of the 12 helix DNA origami structure demonstrating different labelling strategies. (b) Widefield image showing DNA origami structures functionalized with TAMRA (green) as a reference and with AlexaFluor 647 (red) attached at handle positions 1, 3 and 7 of the helix 0. (c) Intensity-time traces corresponding to stepwise photobleaching experiments for structures containing single, double and triple fluorophore occupancy. (d) Left: Dual-color STORM image showing DNA origami functionalized with AlexaFluor 647 (red) and TAMRA (green), inset shows the STORM image of AlexaFluor 647 alone, without the TAMRA signal for ease of visualization; (Right) Clustering analysis of the AlexaFluor 647 STORM image corresponding to the inset. (e) Distribution for the number of localizations detected for 1 (black), 2 (red) and 3 (cyan) fluorophores (f) Number of localizations for 1, 2 and 3 fluorophores (n=3 experiments). The box shows $25/75^{th}$ percentile, the line is the median value and the whiskers are the standard deviation (see Table 1). (g) Left: Dual-color STORM image showing DNA origami functionalized with TAMRA (green) and Dynein-GFP (GFP immunostained with Alexa Fluor 405/Alexa Fluor 647, red), inset shows the STORM image of AlexaFluor 647 alone; (Right) Clustering analysis of the AlexaFluor 647 STORM image corresponding to the inset. (h) Calibration curve showing the number of localizations for 1, 2 and 3 motors (n=3 experiments). The box shows $25/75^{th}$ percentile, the line is the median value and the whiskers are the standard deviation (see Table 1). (i) The localization distribution fits remarkably well to a convolution of 2 (black), 4 (red) and 6 (blue) log-normal distributions f1 corresponding to the distribution of a single GFP. Scale bar 200nm (d, g), Scale bar 5μm (b).

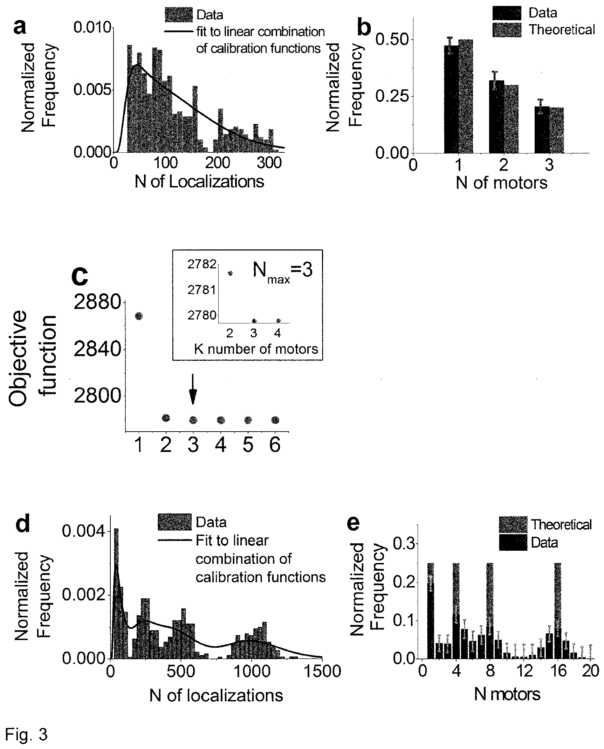

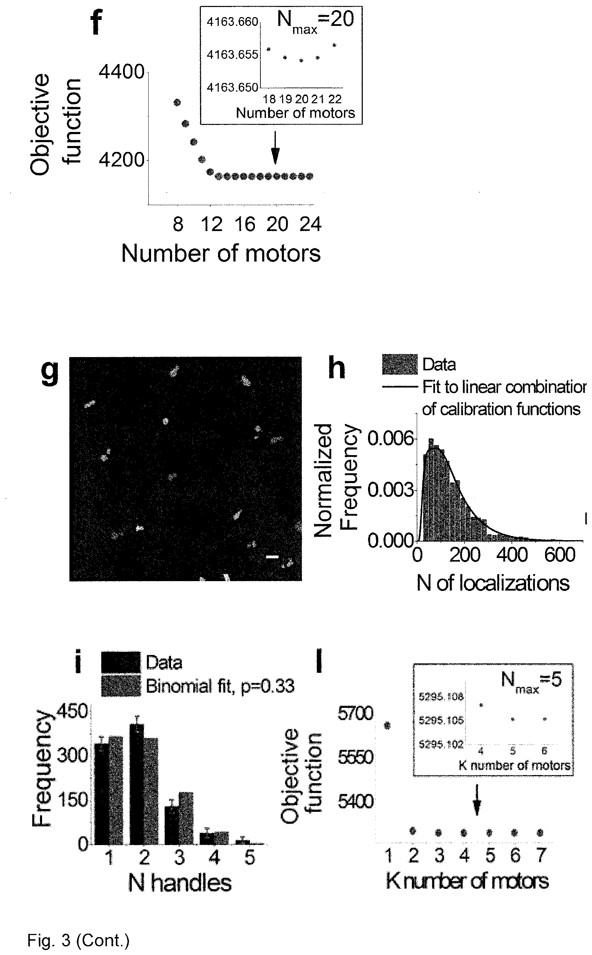

Figure 3 Validation of stoichiometry determination (a) Estimation of the stoichiometry for a synthetic sample with known percentage of single, double and triple motors generated from the DNA-origami images, fit to a linear combination of lognormal distributions (b) and the evolution of the objective function F for a number of stoichiometries with a minimum corresponding to a stoichiometry of 3 motors ($N_{max}$=3) (c) (d) Estimation of the stoichiometry for a synthetic sample with equal percentage (25%) of 1, 4, 8 and 16 motors (2, 8, 16 and 32 GFPs) generated from the DNA-origami images, fit to a linear combination of lognormal distributions (e) and the corresponding objective function F for a number of stoichiometries with a minimum corresponding to a stoichiometry of 20 motors ($N_{max}$=20) (f). (g) Clustering analysis of STORM images for DNA chassis functionalized with 5 motors. Scale bar 200nm. (h) Distribution showing the total number of localizations per 5 dynein motors (red) and the corresponding fit to a linear combination of log normal functions considering up to 5 dimers (black line) (i) The percentage of 1,2,3,4 and 5 motors obtained from the fit in (black) (37% single, 44% two, 14% three, 4% four, 1% five dynein motors) matches to a binomial distribution with a labeling efficiency of p=0.33 (red) (Reduced ChiSquared=0.0009) and (l) the corresponding objective function F for a number of stoichiometries with a minimum corresponding to a stoichiometry of 5 motors ($N_{max}$=5). Errors bars in (b), (e) and (i) refer to the lower bound to the standard errors based on the Fisher Information Matrix.

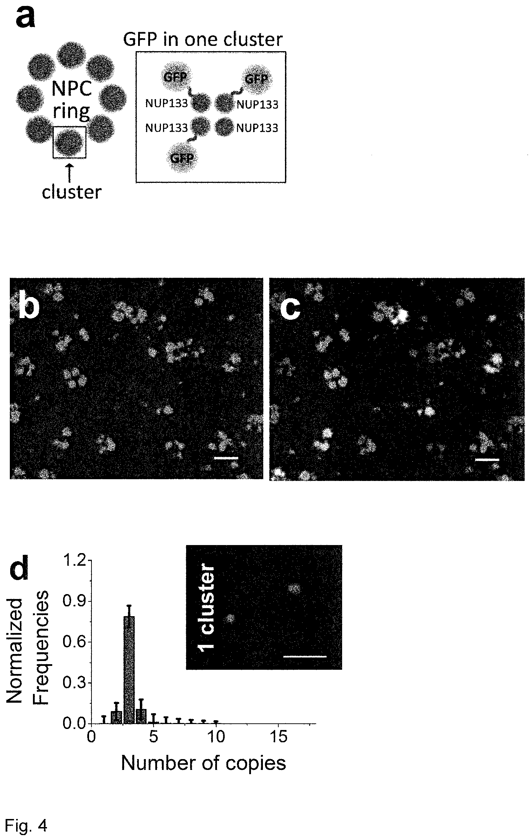

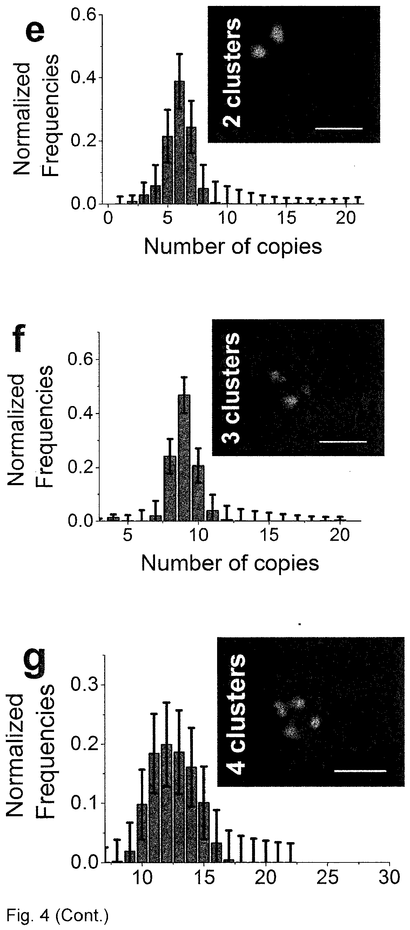

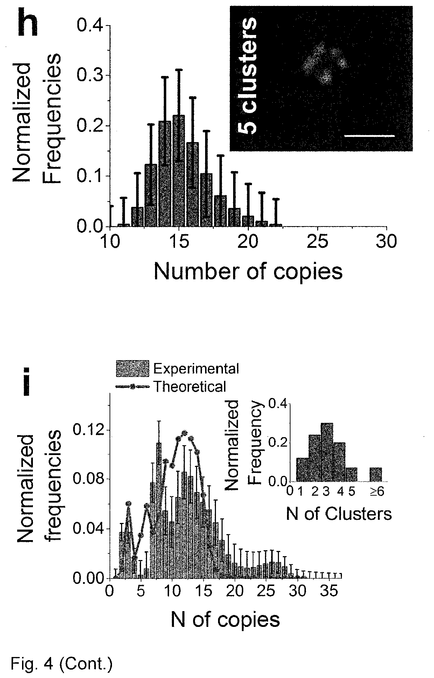

Figure 4. Quantification of NUP133 complexes in U2OS cells: (a) Schematic representation of the NPC and Nup133 subunit composition reflecting the terminology used in the manuscript. (b) STORM image showing NUP133 in siRNA resistant NUP133-GFP expressing U20S cell in which the endogenous copy of NUP133 was knocked down by siRNA. (c) Corresponding clustering analysis of the STORM image. GFP copy-number distribution for NUP133 extracted from the fit of manually sorted data (d-h) corresponding to 1,2,3,4,5 clusters, respectively (d-h insets). GFP copy-number distributions estimated in the whole cell for NUP133 (i, black bars) by fitting the distribution of the number of localizations per NPC to a linear combination of calibration functions considering contributions up to 32 monomers. Distribution of stoichiometries obtained by weighing the sorted data (i, red line) with their occurrence in the super-resolution images (i, inset). Scale bars: 200 nm (b-g). Errors bars in (d-i) refer to the lower bound to the standard errors based on the Fisher Information Matrix.

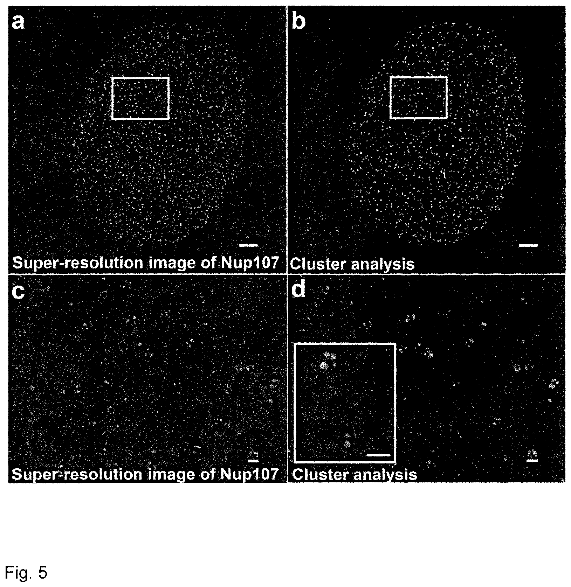

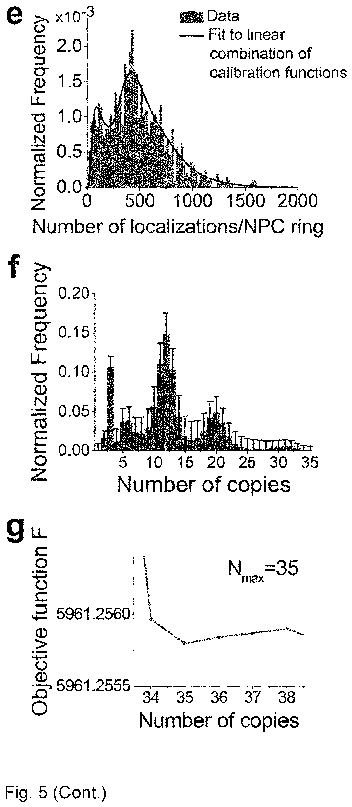

Figure 5. Quantification of Nup107 complexes in U2OS cells: (a, c) STORM image showing Nup107 in siRNA resistant Nup107-GFP expressing U20S cell in which the endogenous copy of Nup107 was knocked down by siRNA: whole nucleus (a) and zoomed region (c). (b, d, inset) Corresponding clustering analysis of the STORM image. (e) Distribution of the number of localizations per NPC (red) and the corresponding fit to a linear combination of calibration functions (black line) (N=1 independent experiment, N= 855 NPC rings analysed). (f) GFP copy-number distribution for Nup107 extracted from the fit minimizing the objective function. Errors bars: lower bound to the standard errors based on the Fisher Information Matrix. Scale bars: 2um (a, b) 200 nm (c, d).

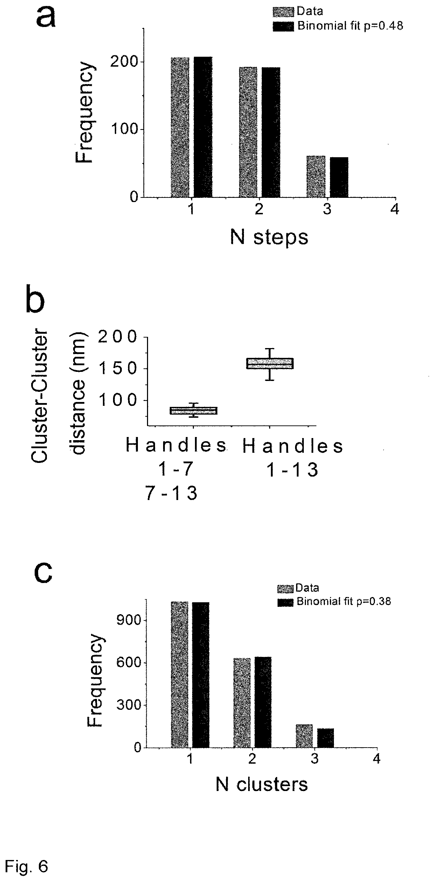

Figure 6. DNA origami characterization: (a) The number of counted steps from the stepwise photobleaching experiments (red) fit to a binomial distribution giving a handle/anti-handle attachment probability of 48% (black) (N=3 independent experiments, $N_{1step}$= 206, $N_{2steps}$= 192, $N_{3steps}$= 61, Reduced ChiSquare=0.0018). (b) After clustering analysis of DNA origami images functionalized with AlexaFluor 647, the mean value of the nearest neighbour distance between clusters (Mean distance 85nm, standard deviation=7nm for distances from handle 1 to handle 7 and from handle 7 and 13) and the distance between the two furthest clusters (Mean distance 157nm, standard deviation=17nm, for distances between handle 1 and 13) were calculated by the distribution of center-to-center distances between each cluster identified (N=2 independent experiments, N= 28 images analysed for handles 1-7/7-13, N= 10 images analysed for handles 1-13). (c) The counted number of single, double and triple clusters detected in STORM images of chassis functionalized with Dynein (red) (labelled with AlexaFluor 405/AlexaFluor 647 through GFP immunostaining) gave a distribution that fit well to a binomial with a labelling probability of 38% (black) (N=3 independent experiments, $N_{1cluster}$= 1030, $N_{2clusters}$= 630, $N_{3clusters}$= 160 Reduced ChiSquare=0.0006).

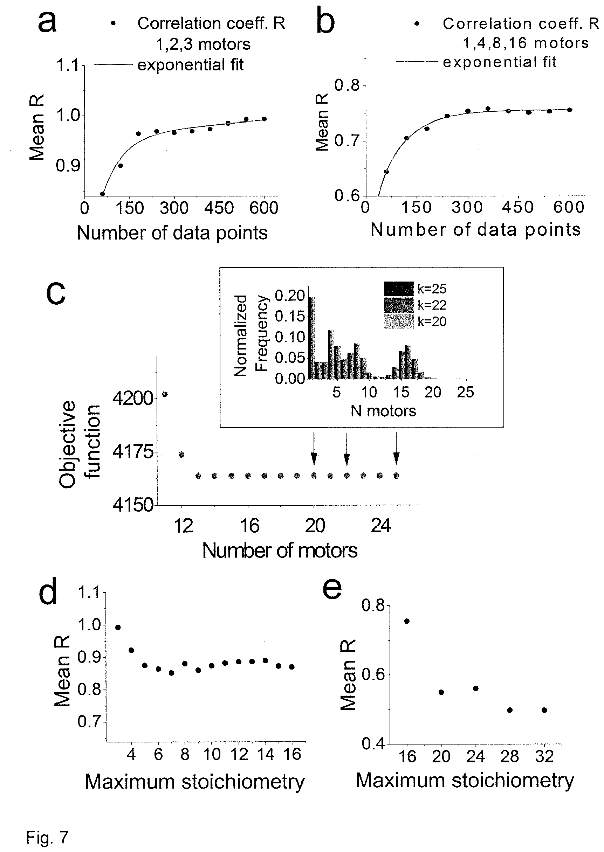

Figure 7. Correlation between estimated and actual values at varying statistics and stoichiometry. (a-b)Pearson correlation coefficient R for known synthetic distributions of localizations corresponding to known fractions of single, double and triple motors ($R_{max}$=0.99 ) (a) and a mixture of 1,4,8,16 motors $R_{max}$=0.75 (b). The distribution used in (a) follows a distribution similar to the one used for 1,2,3 motors shown in Fig2a-c and the values of the fractions are distributed according to an exponential function (y=7.98*exp(-0.47*x). The fractions in (b) are uniformly distributed as the one used for 1,4,8,16 motors in Fig.2d-f. To estimate the mixture of 1,4,8,16 motors the analysis was stopped when the minimum value of the objective function was reached ($N_{max}$=20) (c) and fitting the data to a convolution of more than 20 functions did not change the results (inset). (d) Pearson correlation coefficient R calculated on synthetic distributions of localizations obtained by randomly combining the values measured for single, double and triple motors in order to provide a maximum stoichiometry ranging from 3 to 16 (d). The value of each fraction was calculated according to an exponential function (y=7.98*exp(-0.47*x). (e) Pearson correlation coefficient R calculated on synthetic distributions of localizations obtained by randomly combining the values measured for 1,4,8,16 motors in order to provide increasing stoichiometries ranging from 16 to 32 motors (e). All the fractions have a uniform value.

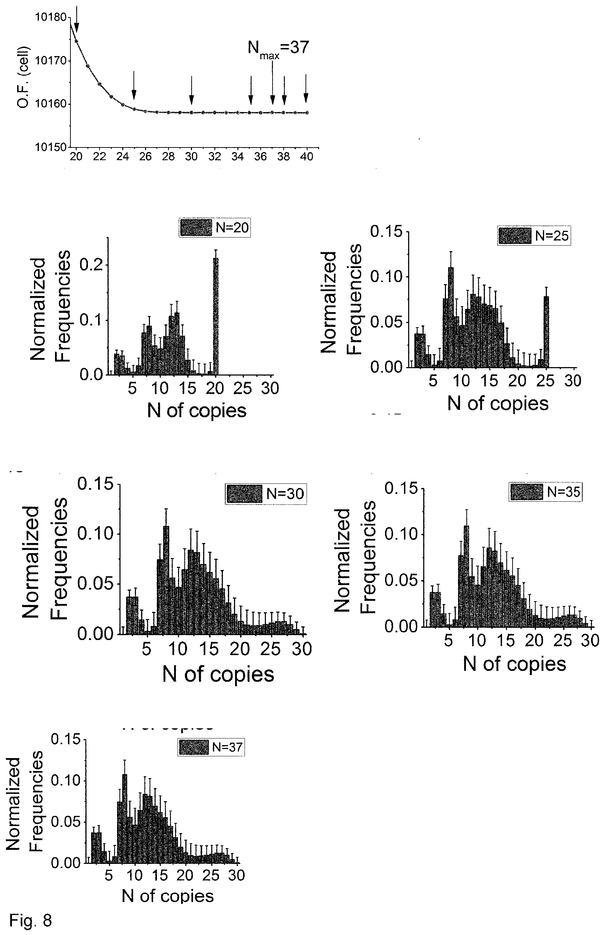

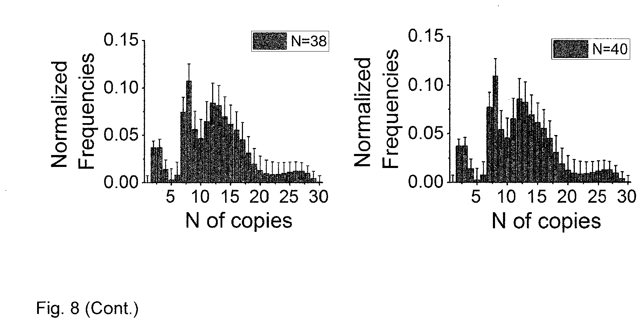

Figure 8. NUP133 distribution obtained by fitting to the convolution of an increasing number of calibration functions. The minimum of the objective function is obtained for $N_{max}$=37 and fitting to a convolution of more than 30 functions did not significantly change the results while fitting to fewer than 30 functions gave rise to isolated peaks at the tail of the stoichiometry distribution.

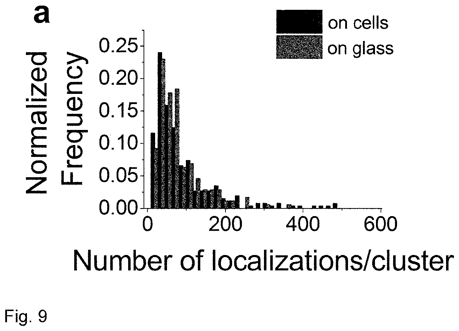

Figure 9. Comparison between DNA origami on different substrates: The number of localizations per cluster was calculated for chassis functionalized with one motor protein (the handle at position 7 was functionalized with Dynein) and immobilized on glass (red) or on BS-C-1 cells (black) through Biotin-Streptavidin attachment. The median value of the number of localizations per cluster distribution for DNA chassis on glass and on cells was (66±56) and (60±89) localizations, respectively (N= 5 independent experiments, N=3077 clusters analysed and N= 4 independent experiments, N=258 clusters analysed on glass and on cells, respectively).

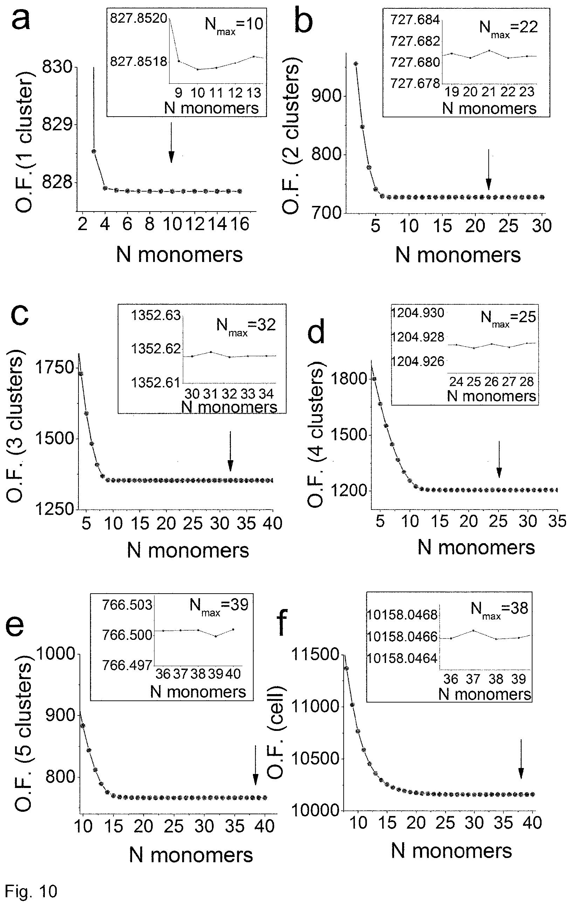

Figure 10. Objective function F values and the stoichiometry ($N_{max}$) at which F is minimized for NUP 133. NPC images were sorted depending upon the number of Nup133 clusters: 1 Cluster (a), 2 Clusters (b), 3 Clusters (c), 4 Clusters (d), 5 Clusters (e) and whole cell (f).

## Detailed Description of the Invention

The inventors have developed a method for quantifying protein copy number in fluorescence based super-resolution microscopy using DNA origami. This calibration method is suitable to quantify the average protein copy number in a cell and to determine the abundance of various oligomeric states.

### Method for obtaining a calibration curve for quantifying protein copy number in fluorescence-based super resolution microscopy

In an aspect, the invention relates to a method for obtaining a calibration curve for quantifying protein copy number in fluorescence-based super resolution microscopy which comprises

a) incubating a DNA origami immobilized on a support, wherein the DNA origami comprises handle oligonucleotides protruding from said DNA origami, said handle oligonucleotides being attached to the DNA origami at predetermined positions and at least one tag, with a protein of interest functionalized with oligonucleotides complementary to the handles protruding from said DNA origami, in conditions allowing the hybridization between the oligonucleotides attached to the DNA origami and the oligonucleotides attached to the protein of interest,

b) recording a super resolution image of the protein of interest which colocalizes with the tag of the DNA origami,

c) clustering the image obtained in step b) and identifying the clusters separated by the distance between the handles to obtain the number clusters in said image obtained in step b),

d) fitting a generic probability distribution function depending on a set of parameters $\mu$ to the distribution of the number of localizations $x$ for one predetermined cluster,

$$f_1(\mu;\ x)$$

and extending it iteratively to larger clusters by using the equation for n= 2, 3...N$_{max}$

$$f_n = f_{n-1} \otimes f_1$$

where $\otimes$ represent the convolution in respect to the x variables between two functions and Nmax is a predetermined maximum number of clusters, and

e) obtaining a calibration curve by the parameters determined through the fitting procedure described in d), wherein steps d) and e) are executed by a computer and wherein the super resolution image of the protein of interest is obtained by detecting said protein with a fluorescent antibody, fluorescent nanobody or fluorescent halo/snap tag specific for the protein of interest.

According to the method of the invention any distribution function can be used. As a way of illustrative non limitative example the distribution function f1 is

$$f_1(x) = \frac{1}{x\mu_2\sqrt{2\pi}} e^{-\frac{(\ln x - \mu_1)^2}{2\mu_2{}^2}}$$

Values $\mu_1$ and $\mu_2$ are two free parameters which are determined through fitting the $f_1$ function to the distribution of localizations obtained from one cluster corresponding to one "protein X" in the calibration experiment.

"Calibration curve", as used herein," is a calibration standard that can be used to quantify protein copy number from super-resolution images obtained after immunofluorescence labeling. In particular, it can be used to extract average protein copy-numbers in a given image by comparing the median number of localizations obtained in the cellular context to the curve.

"Fluorescence-based super resolution microscopy", as used herein relates to a microscopic technique which allows obtaining an image with an axial and lateral resolution under 100 nm allowing single molecule localization.

In a preferred embodiment, the images obtained are characterized by a lateral (XY) resolution of approximately 20-30 nm and axial (Z) resolution of 50-60 nm.

The super resolution images can be obtained by any super resolution technique known in the art. Super-resolution techniques allow the capture of images with a higher resolution than the diffraction limit. They fall into two broad categories, "true" super-resolution techniques, which capture information contained in evanescent waves, and "functional" super-resolution techniques, which use clever experimental techniques and known limitations on the matter being imaged to reconstruct a super-resolution image. There are two major groups of methods for functional super-resolution microscopy:

1. Deterministic super-resolution: The most commonly used emitters in biological microscopy, fluorophores, show a nonlinear response to excitation, and this nonlinear response can be exploited to enhance resolution. These methods include without limitation STED, GSD, RESOLFT and SSIM.

2. Stochastical super-resolution: The chemical complexity of many molecular light sources gives them a complex temporal behaviour, which can be used to make several close-by fluorophores emit light at separate times and thereby become resolvable in time. These methods include without limitation SOFI and all single-molecule localization methods (SMLM) such as SPDM, SPDMphymod, PALM, FPALM, STORM and dSTORM.

In a preferred embodiment, the super resolution image is obtained by a stochastical super resolution technique, preferably STORM, PALM and fPALM, and more preferably by STORM. STORM combines two concepts: single molecule localization and fluorophore photoswitching. The first concept allows one to localize the position of a single fluorophore with nanometer precision. Photoswitching makes it possible to "turn off" most fluorophores into a dark state and "turn on" only a small subset of them at a time. As a result, the images of the "active" fluorophores are isolated in space and their positions can be localized with high precision. Once all the fluorophores are imaged and their positions are localized, a high-resolution image can be reconstructed from these localizations. To date, the spatial resolution achieved by this technique is ~20 nm in the lateral dimensions and ~50 nm in the axial dimension. More details of STORM technology are described in WO2013090360, WO2009085218 and EP2378343.

In step a) of the first method of the invention, a DNA origami immobilized on a support, wherein the DNA origami comprises handle oligonucleotides protruding from said DNA origami and at least one tag, said handle oligonucleotides being attached to the DNA origami at predetermined positions, is incubated with a protein of interest functionalized with oligonucleotides complementary to the handles protruding from said DNA origami, in conditions allowing the hybridization between the oligonucleotides attached to the DNA origami and the oligonucleotides attached to the protein of interest,.

"DNA origami" as used herein relates to the nanoscale folding of DNA to create non-arbitrary two- and three-dimensional shapes at the nanoscale. The specificity of the interactions between complementary base pairs makes DNA a useful construction material, through design of its base sequences. DNA is a well-understood material that is suitable for creating scaffolds that hold other molecules in place or to create structures all on its own.

In general, the DNA origami process involves the folding of one or more long, "scaffold" or "chassis" of DNA strands into a particular shape using a plurality of rationally designed "staple" DNA strands. The sequences of the staple strands are designed such that they hybridize to particular portions of the scaffold strands and, in doing so, force the scaffold strands into a particular shape. This chassis serves as a skeleton for attaching additional components via the use of "handle" sequences that project outward. These handles provide site- and sequence-specific attachment points for single fluorophores as well as proteins of interest and allow testing of several different labeling strategies. Such strategies involve fluorescent antibody, fluorescent nanobody or fluorescent halo/snap tag..

Methods useful in making of DNA origami structures are known by those skilled in the art. In some embodiments, the DNA origami device (or "robot" or "DNA robot" or "DNA nanorobot") may include a scaffold strand and a plurality of rationally designed staple strands. The scaffold strand can have any sufficiently non-repetitive sequence. The sequences of the staple strands are selected such that the DNA origami device has at least one shape in which biologically active moieties can be sequestered.

In some embodiments, the DNA origami can be of any shape that has at least one inner surface and at least one outer surface. In general, an inner surface is any surface area of the DNA origami device that is sterically precluded from interacting with the surface of a cell, while an outer surface is any surface area of the DNA origami device that is not sterically precluded from interacting with the surface of a cell. In some embodiments, the DNA origami device has one or more openings (e.g., two openings), such that an inner surface of the DNA origami device can be accessed by sub-cellular sized particles. In another particular embodiment, the DNA origami can comprise several double helices, by way of example the DNA origami can comprises 6 parallel double helices, 8 parallel double helices, 10 parallel double helices, 12 parallel double helices, 14 parallel double helices. In a more preferred embodiment, the DNA origami comprises 12 parallel double helices. In a preferred embodiment, the DNA origami comprises 6 inner helices and 6 outer helices.

In a preferred embodiment, the DNA origami chassis is the one described in Derr et al,. Science 338, 662-665 (2012) or Goodman, B. S. et al., Meth. Enzymol. 540, 169-188 (2014).

As a way of illustrative non- limitative example the DNA origami can be prepared using p8064 scaffold and oligonucleotide staple sequence by folding the 12-helix bundle DNA origami chassis structures in DNA origami folding buffer by way of illustrative non-limitative example (5 mM Tris [pH 8.0], 1 mM EDTA and 16 mM MgCl2) by mixing 100 nM p8064 scaffold with 600 nM core staples, 3.6 $\mu$M handle staples, 3.6 $\mu$M biotin staples, and 9 $\mu$M fluorophore anti-handles. In addition a thermal folding cycle is run, by rapid heating to 80°C and cooling in single degree increments to 65°C for 75 min, followed by cooling in single degree increments to 30°C for 17.5 hr. The folded DNA origami chassis can be stored at either 4°C or -20°C.

Alternatively the DNA origami chassis is commercially available.

The DNA origami used in the present invention is immobilized on a support. "Support", as used herein relates to any surface wherein the DNA origami can be attached. As a way of illustrative non limitative examples, a support is a coverslip. Methods for immobilizing DNA origami structures on a solid substrate are not particularly limited and are known in the art. They include for example top-down patterning approaches such as ink-jet printing, DPN, polymer pen lithography and the like.

The DNA origami comprises handle oligonucleotides protruding from said DNA origami and at least one tag.

"Handle oligonucleotides", as used herein relates to sequence that project outward from the structure of the DNA and provide site and sequence specific attachment points from single fluorophores, proteins of interest and allow testing several different labelling strategies.

"Tag", as used herein relates to any molecule which allows the identification by super resolution technique of the DNA origami . In a preferred embodiment, the tag is a fluorescence tag, more preferably TAMRA. When applied to the protein of interest, said tag, named as second tag, is a peptide sequence which can be used to identify said protein of interest and that forms part of a fusion protein together with the protein of interest.

Preferably, the DNA origami nanostructures used in the method of the present invention carry one or more tags. Tags can be located anywhere on the DNA origami structure. In a particular embodiment, the tag is presented into the direction away from the solid substrate in the final assembly. Said tags are preferably selected from the group consisting of metal nanoparticles, semiconductor nanoparticles, proteins, peptides, nucleic acids, lipids, polysaccharides, small molecule organic compounds, colloids, and combinations thereof. Methods for attaching various types of tags to DNA origami structures are not particularly limited and are known in the art. Tags can be attached directly or indirectly. Indirect attachment can be effected by suitable binding pairs known in the art, e.g. Streptavidin-biotin, self-ligating linker proteins such as SNAP- or Halo-Tag, and antibodies, antibody mimetics or antibody fragments binding to their respective antigens. In the context of antibody fragments, single-chain antibody fragments (scFv) are particularly preferred. Each of these binding agents can be present on the DNA origami structure, with the respective binding partner present on the tags. Means for coupling said binding agents to the DNA origami structure and the tags are known in the art. Methods for the direct attachment of tags to DNA origami structures include expressed protein ligation, chemoenzymatic coupling (e.g. Sortase A coupling), coiled-coil peptide assembly, and the generation of oligonucleotide conjugates. Further, conventional homo- and heterospecific cross-linkers containing reactive groups directed against carboxyls, amines, thiols or orthogonal coupling pairs such as azide/alkyne cycloaddition or variants thereof can be used.

In a preferred embodiment, a tag is bound to the DNA origami through a sequence complementary to an oligonucleotide protruding from the DNA origami. In a preferred embodiment the tag is a fluorescence tag, and more preferably TAMRA.

The DNA origami can comprise several tags at any position. In a preferred embodiment, the DNA origami comprises at least 1, at least 2, at least 3, at least 4, at least 5 tags, at least 6, at least 7, at least 8, at least 9, at least 10, at least 11 or more tags. In a more preferred embodiment, the tag is localized at position 14 of each of the outer helices.

Protein of interest as used herein relates to any protein. In a preferred embodiment the protein of interest forms part of a fusion protein together with any other protein such as a second tag.

Said tag is generally a peptide or amino acid sequence which can be used in the isolation, purification or detection of said fusion protein. Illustrative non-limitative examples of tags are histidine tag (His-tag or HT), FLAg tag, GFP Arg-tag, FLAG-tag Strep-tag, an epitope capable of being recognized by an antibody, such as c-myc-tag, HA tag, V5 tag SBP-tag, S-tag, calmodulin binding peptide, cellulose binding domain, chitin binding domain, glutathione S-transferase-tag, maltose binding protein, NusA, TrxA, DsbA, Avi-tag, etc.

In a preferred embodiment, the protein of interest is forming a fusion protein together with GFP.

In addition, a protein of interest must be functionalized with oligonucleotides complementary to the handles protruding from the DNA origami chassis. In a preferred embodiment, the protein of interest is functionalized with oligonucleotides complementary to the handles protruding at any position 0 to 14, particularly at positions 1, 7 and 13 of helix 0 of the DNA origami.

The person skilled in the art knows several methods to functionalize a protein with oligonucleotides, such as those disclosed in the experimental part of the present invention.

A skilled person in the art knows the conditions allowing the hybridization between the handles protruding oligonucleotides of the DNA origami and the oligonucleotides of the protein of interest

As anybody skilled in the art knows, "conditions allowing hybridization" according to the method of the present invention are such that (i) do not compromise the structure and integrity of the DNA origami structures, the first and second structural features, e.g. ssDNA strands, and the solid substrate, and (ii) allow for binding of the first and second structural features, e.g. for the Watson-Crick pairing between the protruding ssDNA strands of the origami structures and the ssDNA capture strands, so that immobilization of the origami structures on the solid substrate is achieved.

In particular embodiment, the hybridization is performed under stringent conditions.

The phrase "stringent hybridization conditions" refers to conditions under which a probe will hybridize to its target subsequence, typically in a complex mixture of nucleic acid, but to no other sequences.

Stringent conditions are sequence-dependent and will be different in different circumstances. Longer sequences hybridize specifically at higher temperatures. See Tijssen S, "Overview of principles of hybridization and the strategy of nucleic acid assays", Laboratory Techniques in Biochemistry and Molecular Biology (Elsevier Science Publishers B.V., Amsterdam, The Netherlands 1993). Generally, stringent conditions are selected to be about 5-10°C degrees lower than the thermal melting point (Tm) for the specific sequence at a defined ionic strength pH. The Tm is the temperature (under defined ionic strength, pH, and nucleic concentration) at which 50 percent of the probes complementary to the target hybridize to the target sequence at equilibrium (as the target sequences are present in excess, at Tm 50 percent of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g. 10 to 50 nucleotides) and at least about 60° C for long probes (e.g. greater than 50 nucleotides). Stringent conditions may also be achieved with the addition of destabilizing agents such as formamide. For high stringency hybridization, a positive signal is at least two times background, preferably 10 times background hybridization. Exemplary high stringency or stringent hybridization conditions include: 50 percent formamide, 5xSSC and 1 percent SDS incubated at 42°C or 5xSSC and percent SDS incubated at 65°C, with a wash in 0.2xSSC and 0.1 percent SDS at 65°C.

Step b) of the first method of the invention comprises recording a super resolution image of the protein of interest which colocalizes with the tag of the DNA origami.

"Super resolution image" as used herein, refers to an image with an axial and/or lateral resolution below the diffraction limit, for example under 100 nm allowing single molecule localization.

Recording an image by means of an optical sensor aiming to the optically excited sample provides a bitmap image at certain resolution having information about the protein localization organization. Once all the fluorophores are imaged and their positions are localized, a high-resolution image can be reconstructed from these localizations. This information will be used to provide characteristic length scales and density of some relevant structural parts of the protein that allows identifying clusters of the protein of interest, particularly a fluorescent probe.

"Colocalization" as used herein, relates to the observation of the spatial overlap between two different signals, one from the protein of interest and the other from the DNA origami.

As a skilled person in the art can understand in order to obtained a super resolution image of the protein of interest is necessary the labeling of the protein of interest with a molecule that allows detecting a signal from said protein, for example antibody, nanobody, Halo, SNAP, clip substrate, depending on the labeling strategy used but having a molecule that can be detected by super-resolution microscopy. According to the invention, the super resolution image of the protein of interest is obtained by detecting said protein with a fluorescent antibody, fluorescent nanobody or fluorescent halo/snap tag specific for the protein of interest.

By way of illustrative non limitative example the protein of interest may be detected by a first antibody against an epitope of the protein of interest or against a sequence expresses together with the protein of interest in a fusion protein, and detecting the protein of interest: antibody binding with a secondary antibody, having at least one photoswitchable fluorophore adapted to be optically excited at a certain wavelength $\lambda_1$ and to emit light at a wavelength $\lambda_2$ different from $\lambda_1$. When the sample having the antibody:protein of interest complex is excited with optical energy, for instance by means of a laser beam of a wavelength $\lambda_1$, those locations of the antibody: protein of interest complex linked to the photoswitchable fluorophore emit light at the wavelength $\lambda_2$.

Step c) of the first method of the invention comprises clustering the image obtained in step b) and identifying the clusters separated by the distance between the handles to obtain the number clusters in said image obtained in step b),

The individual locations of a fluorophore and cluster information need to be identified over the image.

Analysis and reconstruction of super resolution images may be performed by obtaining fluorescent probe positions. Several known softwares can be used for obtaining fluorescent probe positions, as illustrative non-limiting example the Insight 3 provided by BO Huang, University of California, San Francisco. Molecules may be identified by a threshold and the radial positions x and y are extracted by fitting with a simple Gaussian function. As a way of illustrative, non-limitative examples, the final image is obtained plotting each identified molecule as a Gaussian spot with a width corresponding to the localization precision (9nm) and finally corrected for drift. Molecules appearing within a distance of 9nm are merged and considered as the same molecule. Spatial clusters of localizations were identified based on a distance-based clustering algorithm, by means of custom-written code written in Matlab11. The localizations list may be first binned to 20 nm pixel size images that were filtered with a square kernel ($7 \times 7$ pixels2) and thresholded to obtain a binary mask. Specifically, a density map was built by 2-dimensional convolution of the localization images with a square kernel ($7 \times 7$ pixels2) and a constant threshold was used to digitize the maps into binary images. The low-density areas, where the density is lower than the threshold value and a value of 0 was assigned, are discarded from further analysis. Only the components of the binary image, where adjacent (6-connected neighbors) non-zero pixels were found, are analysed. A peak finding routine provides the clusters number and the relative centroid coordinates from the maxima of the density map in the connected regions. Molecular localizations lying over connected regions of the mask were assigned to each cluster using a distance-based algorithm, depending on their proximity to the cluster centroids. For each cluster, its centroid position is iteratively re-calculated and saved for further analysis until convergence of the sum of the squared distances between localizations and the associated cluster is reached. It may be obtained the cluster centroid positions, the number of localizations obtained per cluster and the cluster size.

In the particular case that 2 molecules of protein of interest are attached to one DNA origami, then single clusters and double clusters will be obtained. In the particular case that 3 molecules of protein of interest are attached to one DNA origami, then single, double and triple clusters will be obtained.

As the person skilled in the art can understand, in order to perform the method for obtaining a calibration curve according to the invention, several previous steps can be performed. By way of example, methods for determining the efficiency of the anti-handle oligos to the complementary handle oligos in the DNA origami. Said determination can be performed as described in the experimental part. In addition, the number of tags successfully conjugated to the DNA origami can be determined by way of illustrative, non-limitative example by single-step photobleaching or STORM.

In a preferred embodiment, the clusters analyzed in step c) are separated by a distance shorter than 200 nm, such as between $85 \pm 7$nm and $157 \pm 17$ nm.

Step d) of the first method of the invention comprises fitting a generic probability distribution function depending on a set of parameters $\mu$ to the distribution of the number of localizations $x$ for one predetermined cluster,

$$f_1(\mu; x)$$

and extending it iteratively to larger clusters by using the equation for n= 2, 3...$N_{max}$

$$f_n = f_{n-1} \otimes f_1$$

where $\otimes$ represent the convolution in respect to the x variables between two functions and $N_{max}$ is a predetermined maximum number of clusters

It is understand that a statistical parameter of the number of localizations for at least one cluster may be determined previous to step d). In a preferred embodiment, the statistical parameter is obtained for the number of localizations for two cluster, three clusters or more. Alternatively, the distribution function may be obtained from the fit parameters

"Localization" as used herein relates to the centroid of the pixels defining a cluster.

The term "statistical parameter" relates to a quantity that indexes a family of probability distributions. In a preferred embodiment, the statistical parameter is selected from the group consisting of median, mean, percentile or combinations thereof.

In a preferred embodiment, Nmax is the number for which the objective function is minimum, however the shape of the stoichiometry distribution obtained after the fit can also help guide the choice for Nmax as its tail should show a smooth decay.

The "probability distribution" as used herein is a description of a random phenomenon in terms of the probabilities of events. Examples of random phenomena can include the results of an experiment or survey. A probability distribution is defined in terms of an underlying sample space, which is the set of all possible outcomes of the random phenomenon being observed. The sample space may be the set of real numbers or a higher-dimensional vector space, or it may be a list of non-numerical values. A "probability distribution function" is some function that may be used to define a particular probability distribution. As used herein, a "function" is a relation between a set of inputs and a set of permissible outputs with the property that each input is related to one output.

Some aspects of the present disclosure relate to fitting functions. A "fitting function," as used herein, refers to a mathematical function used to fit the number of localizations distribution. An example of fitting function for use as provided herein include, without limitation, a log normal distribution. The above-described embodiments of the present disclosure can be implemented in any of numerous ways. For example, the embodiments may be implemented using hardware, software or a combination thereof. When implemented in software, the software code can be executed on any suitable processor or collection of processors, whether provided in a single computer or distributed among multiple computers. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g. , one or more processors) that is programmed using microcode or software to perform the functions recited above. In this respect, it should be appreciated that one implementation of the embodiments of the present disclosure comprises at least one non-transitory computer-readable storage medium (e.g. , a computer memory, a floppy disk, a compact disk, a tape, etc.) encoded with a computer program (i.e., a plurality of instructions), which, when executed on a processor, performs the above-discussed functions of the embodiments of the present disclosure. The computer-readable storage medium can be transportable such that the program stored thereon can be loaded onto any computer resource to implement the aspects of the present disclosure discussed herein. In addition, it should be appreciated that the reference to a computer program which, when executed, performs the above-discussed functions, is not limited to an application program running on a host computer. Rather, the term computer program is used herein in a generic sense to reference any type of computer code (e.g., software or microcode) that can be employed to program a processor to implement the above-discussed aspects of the present disclosure.

Step e) of the first method of the invention comprises obtaining a calibration curve by the parameters determined through the fitting procedure described in d).

All the terms and embodiments described in the present invention are also applicable to this aspect of the invention,

Method for quantifying protein copy number in a sample imaged with super resolution microscopy

In another aspect, the invention relates to a method for quantifying protein copy number in a sample imaged with super resolution microscopy which comprises, obtaining a statistical parameter of the number of localizations in a sample having the protein of interest and comparing it with the calibration curve obtained for said protein of interest according to the method for obtaining a calibration curve of the invention.

In a particular aspect, the sample is imaged by immunofluorescence.

According to the method of the present invention the value of $\sigma$ and $\mu$ are obtained from the calibration curve and they are used in the method for quantifying protein copy number.

In a preferred embodiment, the statistical parameter is selected from the group consisting of median, mean or any other statistical parameter.

Quantifying, as used herein, refers to determine protein number and stoichiometry. As will be understood by those skilled in the art, the quantification, although preferred to be, need not be correct for 100% of the samples to be detected or evaluated. The term, however, requires that a statistically significant portion of number of proteins can be determined. Whether a number of proteins is statistically significant can be determined by a skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

"Sample", as used herein refers to any sample susceptible of containing proteins, and it can be obtained by conventional methods known by those of average skill in the art, depending on the nature of the sample.

In a particular embodiment, said sample is a biopsy sample, tissue, cell or biofluid sample (plasma, serum, saliva, semen, sputum, cerebral spinal fluid (CSF), tears, mucus, sweat, milk, brain extracts and the like). Said samples can be obtained by any conventional method. In another aspect, the sample is a cell culture sample.

In a particular aspect, the sample is imaged by immunofluorescence.

In a preferred embodiment, the super resolution image is obtained by a stochastical super resolution technique, preferably STORM, PALM and fPALM, and more preferably by STORM.

In another particular embodiment, the DNA origami can comprise various double helices, by way of example 12 parallel double helices and/the DNA origami comprises ine tag at position 14 of each of the outer helices.

In a preferred embodiment, the protein of interest is functionalized with oligonucleotides complementary to the handles protruding at any position, for example from 0 to 14, particularly at positions 1, 7 and 13 of helix 0.

In a preferred embodiment, the clusters analyzed in step c) are separated by a distance shorter than 200 nm, such as between $85\pm7$ nm and $157\pm17$ nm.

According to the invention, the super resolution image of the protein of interest is obtained by detecting said protein with a fluorescent antibody, fluorescent nanobody or fluorescent halo/snap tag specific for the protein of interest.

All the terms and embodiments previously described are equally applicable to this aspect of the invention.

Method for determining the percentage of oligomeric state of a protein in a sample imaged with super-resolution microscopy

In another aspect, the invention relates to a method for determining the percentage of oligomeric state of a protein in a sample imaged with super-resolution microscopy which comprises fitting the overall distribution of the number of localizations obtained in the sample to

$$g(x) = \sum_{n=1}^{N_{max}} \alpha_n f_n(\mu; x)$$

where $\alpha_n$ represents the weight of the distribution of $n$-mers being $\sum_{n=1}^{N_{max}} \alpha_n = 1$ and $f_n$ is a linear combination of calibration distributions obtained as convoluting function $f_1$, n-times according to:

$$f_n = f_{n-1} \otimes f_1$$

obtained for said protein of interest according to the method for obtaining a calibration curve of the invention, wherein fittings are performed by optimization of an objective function.

By way of illustrative, non-limitative example fittings are performed by a two-step numerical minimization of the objective function:

$$F = -w_L \sum_x p(x) \ln g(x) + w_E \sum_n \alpha_n \ln \alpha_n,$$

which represents the sum of the negative log-likelihood and the entropy. In the first term, $p(x)$ corresponds to the number of occurrences for number of localization $x$. In the first optimization step, it can set $w_L = 1$ and $w_E = \dfrac{\max(x) - \min(x)}{\langle x \rangle}$, with $\langle x \rangle$ representing the average value of the data, and let the optimization run at varying $N_{max}$ until the minimum of the objective function $F_{min}$ is found. By means of this procedure, the maximum number of log-likelihood functions necessary to satisfactorily fit the data is calculated. Once this number is determined, the fit is further optimized by performing a second step of optimization, where the weight of the log-likelihood is set to the inverse of its target value $w_L = 1/F_{min}$.

Oligomeric state as used herein relates to the formation of a macromolecular complex formed by non-covalent bonding of a few proteins. Dimers, trimers, and tetramers are, for instance, oligomers composed of two, three and four monomers, respectively.

"Objective function", as used herein, relates to an equation to be optimized given certain constraints and with variables that need to be minimized or maximized using nonlinear programming techniques. The objective function indicates how much each variable contributes to the value to be optimized in the problem. In a preferred embodiment, the objective function is likelihood, entropy or any combination thereof.

All the terms and embodiments previously described are equally applicable to this aspect of the invention.

Computer program

In another disclosure, the document relates to a computer program comprising instructions which, when the program is executed by a computer, cause the computer to carry out step d)-e) of the method for obtaining a calibration curve for quantifying protein copy number in fluorescence-based super resolution microscopy, to carry out the method for quantifying protein copy number in a sample imaged with super resolution microscopy and/or for determining the percentage of oligomeric state of a protein in a sample imaged with super-resolution microscopy

Computer program code for carrying out operations for aspects of the present document may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

All the terms and embodiments previously described are equally applicable to the present case.

Kit and uses thereof

In another aspect, the invention relates to a kit comprising

a) a DNA origami attachable to a support comprising handle sequences protruding from said DNA and at least one tag, optionally the DNA origami is protected from degradation
b) reagents suitable for obtaining a super resolution image of a protein of interest, wherein the reagents for obtaining a super resolution image of a protein of interest comprises

i) an antibody or nanobody specific for the protein of interest having at least one fluorophore, or
ii) a primary antibody specific for the protein of interest and a secondary antibody having at least one fluorophore and

c) a computer-readable medium comprising instructions which, when executed by a computer, cause the computer to carry out the methods of the invention.

In the context of the present invention, "kit" is understood as a product containing the different reagents necessary for carrying out the methods of the invention packed so as to allow their transport and storage. Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instruction.

In a preferred embodiment, the reagents comprise at least 10%, at least 20%, at least 30%, at least 40%, at least 50%, at least 60%, at least 70%, at least 80%, at least 90% or at least 100% of the total amount of reagents forming the kit.

In a preferred embodiment, if the DNA origami is not attached to a support, the DNA origami is protected from degradation. By way of illustration and non-limitative the DNA origami may be covered with a polymer to preserve from degradation.

The reagents for obtaining a super resolution image of a protein of interest comprise

a) an antibody or nanobody specific for the protein of interest having at least one fluorophore, or
b) a primary antibody specific for the protein of interest and a secondary antibody having at least one fluorophore.

In a more preferred embodiment, said fluorophore is a photoswitchable fluorophore.

In another preferred embodiment, the DNA origami is attached to a support, more preferably to a cover slip.

In another preferred embodiment, the kit comprises additional reagents such as imaging buffer.

In another preferred embodiment, the kit DNA origami comprises 12 parallel DNA double helices. In another preferred embodiment the tag of the DNA origami is localized at potion 14 of each of the outer helices.

As used herein, the term "antibody" refers to immunoglobulin molecules and immunologically active portions of immunoglobulin molecules, i.e., molecules containing an antigen fixing site binding specifically (immunoreacting) with an antigen, such as a protein for example. There are 5 isotypes or main classes of immunoglobulins: immunoglobulin M (IgM), immunoglobulin D (IgD), immunoglobulin G (IgG), immunoglobulin A (IgA) and immunoglobulin E (IgE).

The antibodies that are going to be used in the present invention can be, for example, polyclonal sera, hybridoma supernatants or monoclonal antibodies, antibody fragments, Fv, Fab, Fab' and F(ab')2, scFv, diabodies, triabodies, tetrabodies and humanized antibodies.

The suitable conditions for the formation of the antibody: protein complex to take place are known by the skilled in the art. If the sample containing cells contains histone proteins, then the corresponding antibody:protein will be formed.

"Fluorophore", as used herein, refers to entities that can emit light of a certain emission wavelength when exposed to a stimulus, for example, an excitation wavelength.

"Photoswitchable" as used herein, relates to an entity which can be switched between different light-emitting or non-emitting states by incident light of different wavelengths. Typically, a "switchable" entity can be identified by one of ordinary skill in the art by determining conditions under which an entity in a first state can emit light when exposed to an excitation wavelength, switching the entity from the first state to the second state, e.g., upon exposure to light of a switching wavelength, then showing that the entity, while in the second state can no longer emit light ( or emits light at a reduced intensity) or emits light at a different wavelength when exposed to the excitation wavelength. Examples of switchable entities are disclosed in WO 2008/091296. As a non-limiting example of a switchable fluorophore, Cy5 can be switched between a fluorescent and a dark state in a controlled and reversible manner by light of different wavelengths, e.g., 633 nm or 657 nm red light can switch or deactivate Cy5 to a stable dark state, while 405 nm or 532 nm light can switch or activate the Cy5 back to the fluorescent state.

In some cases, the fluorophore can be reversibly switched between the two or more states, e.g., upon exposure to the proper stimuli. For example, a first stimuli (e.g., a first wavelength of light) may be used to activate the switchable fluorophore, while a second stimuli (e.g., a second wavelength of light) may be used to deactivate the switchable fluorophore, for instance, to a non-emitting state. Any suitable method may be used to activate the fluorophore. For example, in one embodiment, incident light of a suitable wavelength may be used to activate the entity to emit light, i.e., the entity is photoswitchable. Thus, the photoswitchable fluorophore can be switched between different light-emitting or non-emitting states by incident light, e.g., of different wavelengths. The light may be monochromatic (e.g., produced using a laser) or polychromatic.

In another embodiment, the entity may be activated upon stimulation by electric field and/or magnetic field. In other embodiments, the entity may be activated upon exposure to a suitable chemical environment, e.g., by adjusting the pH, or inducing a reversible chemical reaction involving the entity, etc.

Similarly, any suitable method may be used to deactivate the entity, and the methods of activating and deactivating the entity need not be the same. For instance, the entity may be deactivated upon exposure to incident light of a suitable wavelength, or the entity may be deactivated by waiting a sufficient time.

In some embodiments, the switchable entity includes a first, light-emitting portion (e.g., a fluorophore), and a second portion that activates or "switches" the first portion.

Upon exposure to light, the second fluorophore may activate the first fluorophore a, causing the first fluorophore to emit light. Examples of activator fluorophores include, but are not limited to Alexa Fluor 405 (Invitrogen), Alexa 488 (Invitrogen), Cy2 (GE Healthcare), Cy3 (GE Healthcare), Cy3.5 (GE Healthcare), or Cy5 (GE Healthcare), or other suitable dyes. Examples of light-emitting portions include, but are not limited to, Cy5, Cy5.5 (GE Healthcare), or Cy7 (GE Healthcare), Alexa Fluor 647 (Invitrogen), or other suitable dyes. These may linked together, e.g., covalently, for example, directly, or through a linker, e.g., forming compounds such as, but not limited to, Cy5-Alexa Fluor 405, Cy5-Alexa Fluor 488, Cy5-Cy2, Cy5-Cy3, Cy5-Cy3.5, Cy5.5- Alexa Fluor 405, Cy5.5-Alexa Fluor 488, Cy5.5-Cy2, Cy5.5-Cy3, Cy5.5-Cy3.5, Cy7- Alexa Fluor 405, Cy7-Alexa Fluor 488, Cy7-Cy2, Cy7-Cy3, Cy7-Cy3.5, or Cy7-Cy5. In a more preferred embodiment the first fluorophore (activator) is Alexa 405 and the second fluorophore is Alexa 647.

Any suitable method may be used to link the first, light-emitting fluorophore and the second, activation fluorophore. In some cases, a linker is chosen such that the distance between the first and second fluorophore is sufficiently close to allow the activator fluorophore to activate the light-emitting fluorophore as desired, e.g., whenever the light-emitting fluorophore has been deactivated in some fashion. Typically, the fluorophore will be separated by distances on the order of 500 nm or less, for example, less than about 300 nm, less than about 100 nm, less than about 50 nm, less than about 20 nm, less than about 10 nm, less than about 5 nm, less than about 2 nm, less than about 1 nm, etc. Examples of linkers include, but are not limited to, carbon chains (e.g., alkanes or alkenes), polymer units, or the like.

The switchable entity may comprise a first fluorophore directly bonded to the second fluorophore, or the first and second entity may be connected via a linker or a common entity. Whether a pair of light emitting portion and activator portion produces a suitable switchable entity can be tested by methods known to those of ordinary skills in the art. For example, light of various wavelength can be used to stimulate the pair and emission light from the light-emitting portion can be measured to determine whether the pair makes a suitable switch.

Additional details about fluorophores can be found in WO2009/085218.

"Computer-readable medium", as used herein relates to the computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

In another aspect, the invention relates to the use of a kit of the invention for obtaining a calibration curve for quantifying protein copy number in immunofluorescence-based super resolution microscopy, for quantifying protein copy number in a sample imaged with super resolution microscopy and for determining the percentage of oligomeric state of a protein in a sample imaged with super-resolution microscopy.

All the terms and embodiments previously described are equally applicable to this aspect of the invention.

The invention will be described by way of the following examples, which are to be considered as merely illustrative and not limitative of the scope of the invention.

## Examples

## Materials and methods

## STORM optical setup.

Imaging was performed on an inverted Nikon Eclipse Ti microscope (Nikon Instruments). The excitation module is equipped with four excitation laser lines: 405 nm (100 mW, OBIS Coherent, CA), 488 nm (200 mW, Coherent Sapphire, CA), 561 nm (500 mW MPB Communications, Canada) and 647 nm (500 mW MPB Communications, Canada). The laser beam power was regulated through AOMs (AA Opto Electonics MT80 A1,5 Vis) and different wavelengths were mixed and coupled into the microscope objective through dichroic mirrors. The focus was locked through the Perfect Focus System (Nikon) and imaging was performed on an EmCCD camera (Andor iXon X3 DU-897, Andor Technologies). Fluorescence emitted signal was spectrally filtered by a Quad Band filter (ZT405/488/561/647rpc-UF2, Chroma Technology) and selected by an emission filter (ZET405/488/561/647m-TRF, Chroma). For single molecule detection the emitted light was acquired at 25Hz by an oil immersion objective (Nikon, CFI Apo TIRF 100x, NA 1.49, Oil) providing a corresponding pixel size of 157 nm.

## DNA origami structure assembly.

12-helix bundle DNA origami chassis structures were prepared using p8064 scaffold and oligonucleotide staple sequences as previously described (Derr et al., 2012). Briefly, 100 nM scaffold (Tilibit Nanosystems), was mixed with 600 nM core staples (Life Technologies), 3.6 $\mu$M handle staples (IDT), and 9 $\mu$M TAMRA-labeled fluorophore anti-handles (IDT). Folding was performed in DNA origami folding buffer (5 mM Tris [pH 8.0], 1 mM EDTA and 16 mM MgCl2) with heating to 80°C and cooling in single degree increments to 65°C for 75 min, followed by cooling in single degree increments to 30°C for 17.5 hr. Folded chassis were purified by glycerol gradient sedimentation by centrifugation (Lin, C. et al., 2013) through a 10-45% glycerol gradient in TBE buffer supplemented with 11 mM MgCl2 for 130 min at 242,704g in a SW50.1 rotor (Beckman) at 4°C and collected in fractions. Fractions were evaluated with 2% agarose gel electrophoresis and the fractions containing well-folded monomeric chassis were collected.

The following handle sequences were used for binding either AlexaFluor 647 complementary anti-handles or anti-handle labeled dynein motors. Sequence portion in black is complementary to the scaffold, while underline sequence is complementary to anti-handle:

| Binding site | Sequence |
|---|---|
| | AACTGTTGGGAACGTTCCGGCAA<u>TTCACTACTTACCAC TCTACC</u> (SEQ ID NO: 1) |
| H0, #1 | AGATACATTTCGATTGCCTGAGA<u>TTCACTACTTACCAC TCTACC</u>(SEQ ID NO: 2) |
| H0, #3 | GAGGGTTGATATGCTTTCGAGGT<u>TTCACTACTTACCAC TCTACC</u>(SEQ ID NO:3) |
| H0, #7 | ACAAATTCTTACTAAGAACGCGA<u>TTCACTACTTACCAC TCTACC</u>(SEQ ID NO: 4) |
| H0, #11 | GTCAATAGATAATACCTGAGCAA<u>TTCACTACTTACCAC TCTACC</u>(SEQ ID NO: 5) |
| H0, #13 | |

The following sequences were used for functionalizing the chassis structures with biotin for immobilization on streptavidin functionalized surfaces

| Binding site | Sequence |
|---|---|
| H7, #1 | GTAAGCTTTCAGAGGTGGAGCCG TTTTTT(SEQ ID NO: 6)-biotin |
| H4, #5 | AAGGCTTGCCCTCTCAAATGCTT TTTTTT(SEQ ID NO:7) -biotin |
| H7, #9 | AAAAAATTCATATGGTTTACCAG TTTTTT(SEQ ID NO: 8)-biotin |
| H4, #13 | AGTAACATTATCCAATATATGTG TTTTTT(SEQ ID NO: 9) -biotin |

**Dynein purification and BG oligos functionalization.**

Complementary oligonucleotides A* (NH2-GGTAGAGTGGTAAGTAGTGAA(SEQ ID NO: 10) were incubated with BG-GLA-NHS (NEB) by mixing: 16 $\mu$L A* (2mM), 32 $\mu$L of Hepes ph8.5 (200mM), 8 $\mu$L of BG-NHS (20mM) at room temperature for 30 min. Oligos were filtered by 0.1 ultrafree MC durapore membrane (Millipore) and purified using MicroBioSpin6 colums (BIORAD) previously equilibrated in protein buffer (10mM TRIS ph8, 150mM KCl, 10%v/v Glycerol).

Dynein was purified as previously published (Torreno-Pina, J. A. *et al, 2014)* and labeled with BG-oligos while attached to IgG sepharose beads during the purification (Qiu, W. *et al. 2012).* Briefly, yeasts (RPY1084 [Derr et al, 2012]) were grown overnight (200rpm, 30 °C) in YPD (2% glucose) and cultures poured into YPR (2% raffinose). Yeasts culture was transferred in YP media (supplemented with 8ml 200X adenine and 2% galactose) and kept growing for 24h. Cells were pelleted twice (6,000rpm, 6min, 4°C) and froze at -80° C. Ground cells were diluted in Dynein lysis buffer (30 mM HEPES (pH 7.2), 50 mM KAcetate, 2 mM MgAcetate, 1mM EGTA, 10% glycerol, 1 mM DTT, 0.5 mM Mg-ATP, 1 mM Pefabloc) and spun for 1 hour at 60K rpm at 4°. The supernatant was incubated with equilibrated IgG Sepharose beads and nutated for 1-2 hours at 4°. Once nutation was done, beads were washed twice with 1X TEV buffer (10 mM Tris (pH 8.0), 150 mM KCl, 0.5 mM ATP, 1 mM DTT, 1 mM Pefabloc) and then incubated (20 minutes at RT) with BG-oligonucleotides (20 $\mu$M). After functionalization, beads were washed three times in TEV buffer and incubated in TEV protease (1:100 in TEV buffer) for 1 hour at 16°C with slow rotation. Beads were removed with centrifugal filters and protein concentrated with Amikon 100K and frozen in LiqN2. Concentration of the purified dynein (330nM (**Figure 1**)) was accessed by protein gel electrophoresis: 4-20% Criterion TGX Precast Protein Gels stained with SYPRO® Ruby gel stain following the rapid stain protocol. The inventors used a Precision Plus Protein unstained Standard (Biorad) that offer absolute molecular weight accuracy confirmed by mass spectrometry, with a known amount of protein in each band to allow approximation of protein concentration in your sample. The gel has been imaged using the Molecular Imager Gel Doc™ XR+ System and analyzed using the Image Lab™ Software.

**DNA origami structures: sample preparation.**

A LabTek chamber (No. 1.0, 8 well) was rinsed with KOH (1M) and PBS three times. Coverglass was incubated with 100 $\mu$L of streptavidin (0.5 mg/ml in PBS) for 20' and washed 3 times with PBS. The coverglass was subsequently incubated with 100$\mu$l of BSA-Biotin (0.5 mg/mL in PBS) for 20', extensively washed in PBS, and incubated with fiducial markers (Carboxyl Fluorescent Particles, Yellow, 1% w/v Spherotec SPH-CFP-0252-2, diameter 111nm, diluted 1:25000 in PBS). Blocking of coverglass was performed in blocking buffer containing 10% (wt/vol) BSA (Sigma) in DAB solution (30mM Hepes, 50 mM KAcetate, 2 mM MgAcetate, 1mM EGTA 7.5, 10% glycerol, 1 mM DTT, 1 mM Mg-ATP, 2.5 mg/mL casein) for 10 minutes at room temperature. Biotin labeled DNA structures were then incubated on ice for 30' with oligo-functionalized dynein (300nM), diluted up to 30 pM concentration in blocking buffer and incubated on the coverslip for 5 min. Structures were then washed twice and blocked in blocking buffer for 15 min at 4°C. Immuno-staining was performed by incubation with primary antibody (chicken polyclonal anti GFP, Abcam 13970) diluted 1:2000 in blocking buffer for 1h was performed at 4°C. Samples were rinsed 3 times in blocking buffer and incubated for 1h at 4°C with donkey-anti chicken secondary antibodies (1:50 in blocking buffer) labeled with photoactivatable dye pairs for STORM Alexa Fluor 405-Alexa Fluor 647. For experiments on BSC-1 cells (from ATCC, #CLL-26), cells were plated (30,000 seeding density) on 8-well Lab-tek 1 coverglass chamber (Nunc) and grown under standard conditions and fixed with Methanol-Ethanol (1:1) at 20°C for 2' and incubated for 5' with DNA origami (1 motor attached) and rinsed 3 times in DAB solution.

**Nup107 and Nup133: sample preparation.**

Human osteosarcoma U2OS cells (from ATCC, #HTB-96) were plated (30,000 seeding density) on 8-well LabTek chambered coverglass (Nunc) and grown under standard conditions (DMEM, high glucose, pyruvate (Invitrogen 41966052) supplemented with 10% FBS). U2OS were chosen since they are well performing for transfection and siRNA KD of Nup. For GFP-tagged Nup107 and GFP-tagged Nup133 experiments cells were transfected with the constructs (Szymborska, A. *et al.*, 2013) (plasmid from Jan Ellenberg, EMBL, Heidelberg, pEGFP-Nup107-s 32727res, Euroscarf plasmid ref. P0729 and pmEGFP-Nup133-s31401res, Euroscarf plasmid ref. P30728) using Fugene (FUGENE HD Transfection Reagent, Roche 04709705001). Incorporation into the pore of the GFP-tagged Nup was facilitated by depletion of the endogenous protein, performed by RNA interference, transfecting after 24h the cells with a matching siRNA (Nup107 SiRNA s32727 and Nup133 SiRNA s31401, Thermo Fisher, Silencer Select siRNA s32727 and Silencer Select siRNA s31401. Nup107 and Nup133, 3picomol of siRNA per well was used). After 70h cells were rinsed with PFA 3%, extracted with 0.2% Triton X-100 in PBS for 2 min and fixed with PFA (3%) for 7'. Immunostaining of Nup107-green fluorescent protein (GFP) fusion protein was performed using immunofluorescence as described above. Cells lines were regularly tested for microplasma contamination by PCR based standard methods (ATCC, Universal Mycoplasma Detection Kit, 30-1012K).

**STORM imaging conditions.**

The imaging conditions were kept constant for all the experiments. Imaging was performed using TIRF illumination with an excitation intensity of ~1KW/cm$^2$ for the 647 nm readout laser line and ~ 25W/cm$^2$ using the 405 nm laser line. 85,000 frames at 25Hz frame rate were acquired. For dual color imaging of DNA origami structures, fluorescence signal from TAMRA was acquired with 561nm laser (intensity of ~200W/cm$^2$). STORM imaging buffer was used containing GLOX solution as oxygen scavenging system (40 mg/mL$^{-1}$ Catalase [Sigma], 0.5 mg/ml$^{-1}$ glucose oxidase, 10% Glucose in PBS) and MEA 10 mM (Cysteamine MEA [SigmaAldrich, #30070-50G] in 360mM Tris-HCl).

**Data analysis.**

Analysis and reconstruction of super-resolution images were performed using custom software (Insight3, kindly provided by Bo Huang, University of California) by Gaussian fitting of the single molecules images to calculate the molecular localization coordinates. Molecules are identified by a threshold and the radial positions x and y are extracted by fitting with a simple Gaussian function. The final image is obtained plotting each identified molecule as a Gaussian spot with a width corresponding to the localization precision (9nm) and finally corrected for drift. Molecules appearing within a distance of 9nm are merged and considered as the same molecule. Spatial clusters of localizations were identified based on a distance-based clustering algorithm, by means of custom-written code written in Matlab (Puchner, E. M. et al., 2013). The localizations list was first binned to 20 nm pixel size images that were filtered with a square kernel (7 $\times$ 7 pixels$^2$) and thresholded to obtain a binary mask. Specifically, a density map was built by 2-dimensional convolution of the localization images with a square kernel (7$\times$7 pixels$^2$) and a constant threshold was used to digitize the maps into binary images. The low-density areas, where the density is lower than the threshold value and a value of 0 was assigned, are discarded from further analysis. Only the components of the binary image, where adjacent (6-connected neighbours) non-zero pixels were found, are analysed. A peak finding routine provides the clusters number and the relative centroid coordinates from the maxima of the density map in the connected regions. Molecular localizations lying over connected regions of the mask were assigned to each cluster using a distance-based algorithm, depending on their proximity to the cluster centroids. For each cluster, its centroid position is iteratively re-calculated and saved for further analysis until convergence of the sum of the squared distances between localizations and the associated cluster is reached. The cluster centroid positions, the number of localizations obtained per cluster and the cluster size are saved.

For DNA origami calibration, first dual color cluster analysis allowed the identification of TAMRA signal (used as a reference to identify the DNA origami structures) and dynein clusters attached to the same DNA origami. In order to consider only the signal belonging to motors attached to DNA origami structures, only the clusters with a relative distance shorter than 200 nm between the clusters in the two channels were considered for further analysis. Clusters identifying single, double and triple motors were then sorted depending on the number motors attached. Additional filter was applied to select structures with the expected handle to handle distance ($85\pm7$nm and $157\pm17$nm). To ensure the statistical significance the inventors chose a sample size able to ensure a power value close to 1 (the total number of DNA origami considered was N=3077, N=1153, N=250 for single, double and triple motors, respectively.

The distributions of the number of localizations per cluster obtained for DNA origami structures showing 1, 2 and 3 dyneins (corresponding to 2,4,6 GFPs respectively) were used as a calibration standard. To this aim, the inventors considered that the distribution of the number of localizations for a structure composed by n GFP can be recursively obtained as

$$f_n = f_{n-1} \otimes f_1$$

where $\otimes$ represent the convolution and $f_1$ is a log-normal distribution:

$$f_1(x) = \frac{1}{x\mu_2\sqrt{2\pi}} e^{-\frac{(\ln x - \mu_1)^2}{2\mu_2^2}}$$

The distributions of localizations obtained for 1,2,3 dyneins (n=2,4,6) were simultaneously fitted to the functions $f_2$, $f_4$, $f_6$ obtaining the parameters $\mu1 = 3.35$ and $\mu2 = 0.85$. The same parameters were used for all the other fittings. The log-normal distribution was chosen because, among several tested distributions, it provided the best data model.

For a general distribution of number of localizations, the copy number of a given protein can thus be estimated by fitting the distributions to a linear combination of the "calibration" distributions $f_n$

$$g(x) = \sum_{n=1}^{N_{max}} \alpha_n f_n(x)$$

where $\alpha_n$ represents the weight of the distribution of $n$-mers and $\sum_{n=1}^{N_{max}} \alpha_n = 1$.

To estimate motors attached to the DNA origami chassis the fit was performed considering only dimers (linear combination of distributions $f_n$, with even values n=2,4,6,8,...,2k) given the dimeric nature of the motors containing two copies of GFP per motor, while for NPC estimation the fit was performed considering n monomers (linear combination of distributions $f_n$, with values n=1,2,3,4,...,k).

Fittings are performed by a two-step numerical minimization of the objective function:

$$F = -w_L \sum_x p(x) \ln g(x) + w_E \sum_n \alpha_n \ln \alpha_n,$$

which represents the sum of the negative log-likelihood and the entropy. In the first term, p(x) corresponds to the number of occurrences for number of localization x. In the first optimization step, the inventors set $w_L = 1$ and $w_E = \frac{\max(x)-\min(x)}{\langle x \rangle}$, with $\langle x \rangle$ representing the average value of the data, and let the optimization run at varying $N_{max}$ until the minimum of the objective function $F_{min}$ is found. By means of this procedure, the inventors calculate the maximum number of log-likelihood functions necessary to satisfactorily fit the data. Once this number is determined, the inventors further refine the fit by performing a second step of optimization, where the weight of the log-likelihood is set to the inverse of its target value $w_L = 1/F_{min}$. When fitting distributions involving the linear combination of only dimeric terms (n=2,4,6,...,2k), in the second step of optimization the inventors further allow the parameters $\mu1$ and $\mu2$ to slightly vary constrained to a maximum tolerance of 5%, in order to supply to the reduced number of degrees of freedom. Calculation of the errors on the estimated weights $\alpha_n$ was based on the reciprocal of the diagonal elements of the Fisher information matrix and thus represent a lower bound to the standard error of the estimators.

For Nup133 and Nup107 quantification, clustering analysis is carried out to segment single nuclear pores and the distribution of the number of localizations/NPC ring was filtered considering a minimum average cluster radius of 40 nm. The total number of nuclear pores analysed was N=1460 for Nup133 and N=855 for Nup107.

**Statistics and data analysis.**

The DNA origami data used for calibration are obtained by 5 independent experiments and the total number of DNA origami structures imaged was $N_1$=3077, $N_2$=1153, $N_3$=250 for single, double and triple motors, respectively (Figure 2g-i). In the case of validation experiments using DNA origami functionalized with 5 dynein motors, number of DNA origami chassis imaged was N=934 in N=4 independent experiments (Figure 3g-j).

Sorted data were used to quantify Nup133 (Figure 4 d-h) (N=1 experiment, total number of NPC rings analyzed N=798). Images corresponding to 1,2,3,4,5 clusters were sorted (NPC rings number analyzed $N_1$=153 for 1 cluster, $N_2$=122 for 2 clusters, $N_3$=219 for 3 clusters, $N_4$=187 for 4 clusters, $N_5$=117 for 5 clusters, respectively). For NPC quantification in the whole cell the total number of NPC rings analysed was N=1460 for Nup133 (Figure 4i) and N=855 for Nup107 (Figure 5). Cluster occurrence for NUP133 was estimated from the super-resolution images (N=1 experiments, total number of NPC rings counted N=1764, Figure 4i, inset).

The box plots (Figure 2f, Figure 2h and Figure 6b) show 25/75th percentile, the line is the median value and the whiskers represent the standard deviation.

The inventors performed a ChiSquare test to verify the matching of the data to a binomial distribution in all cases (Figure 3j, Figures 6a and c).

Performances of the method and the correlation between estimated and actual values at varying statistics and stoichiometry have been characterized calculating the Pearson correlation coefficient R (Figure 7a,b,d,e).

The error bars on stoichiometry estimation correspond to the lower bound to the standard errors based on the Fisher Information Matrix (Figure 3 b,e,j, Figure 3 d-i, Figure 8 and Figure 5).

Example 1

To overcome these challenges and thus develop versatile calibration standards that can be used for quantifying protein copy-number in intracellular contexts, the inventors took advantage of DNA origami. Specifically, the inventors used a previously developed 3D DNA origami chassis comprised of 12 parallel DNA double helices. This chassis serves as a skeleton for attaching additional components via the use of "handle" sequences that project outward from the structure (Derr, N. D. *et al.* 2012) These handles provide site- and sequence-specific attachment points for single fluorophores as well as proteins of interest and allow testing of several different labeling strategies such as antibody, nanobody and Halo/SNAP tag labeling (Figure 2a). The inventors first used this structure to attach complimentary anti-handle sequences labeled with a single AlexaFluor647 to the three handles located at positions 1, 7 and 13 of helix 0 and thus establish a baseline for the efficiency of attaching the anti-handle oligos to the complimentary handle oligos in the DNA origami. This handle/anti-handle labelling efficiency should be independent of the fluorophore used and only depend on the sequence of the oligos. A single TAMRA fluorophore attached at position 14 of each of the other outer helices (h3, h4, h7, h8, h11, Figure 2a) was used to identify the DNA origami structures on the glass slide (Figure 2b). To determine the number of fluorophores successfully conjugated to the handles, the inventors performed single-step photobleaching experiments and analyzed the intensity-time traces from AlexaFluor647 spots, only taking into account those that showed co-localization with a TAMRA spot. The traces revealed single, double and triple steps as expected (Figure 2c) and the distribution of the number of counted steps fit to a binomial giving a handle/anti-handle labeling probability of 48% (Figure 6a). Next, the inventors performed STORM imaging and once again analyzed the spots that showed co-localization between the AlexaFluor647 and TAMRA labels (Figure 2d left panel). Super-resolution imaging revealed single, double or triple clusters (Figure 2d inset), in agreement with the single-step photobleaching experiments. The inventors segmented these clusters using a previously developed cluster identification algorithm (Ricci, M. A et al., 2015) (Figure 2d right panel) and found that the nearest neighboring clusters were separated by an average of 85±7 nm (Mean±SD) whereas the furthest two clusters were separated by a distance of 157±17 nm (Figure 6b), matching the expected distance between the individual handles used for the labeling. The number of localizations detected from individual clusters showed a broad distribution (Figure 2e) with the median value of the number of localizations obtained for 1, 2 and 3 fluorophores showing a roughly linear increase with the number of fluorophores as expected (Figure 2f and Table 1).

| | 1 cluster | 2 clusters | 3 clusters |
|---|---|---|---|
| **AlexaFluor 647** | (39.0±19) localizations N=105 | (77±21) localizations N=36 | (107±41) localizations N=24 |

(continued)

|  | 1 cluster | 2 clusters | 3 clusters |
|---|---|---|---|
| Dynein-AlexaFluor405/Alexa Fluor647 | (66±56) localizations N=3077 | (150±78) localizations N=1153 | (211±92) localizations N=250 |

Table 1. Median number of localizations for DNA origami functionalized with fluorophores and dynein. Chassis were functionalized at three positions with a single fluorophore (AlexaFluor 647) and the number of localizations was calculated from origami images containing single, double or triple clusters corresponding to single, double and triple AlexaFluor 647 (median values, standard deviations and number of clusters analysed are reported in 1st row).

Chassis were functionalized at three positions with dynein labelled through GFP immunostaining with AlexaFluor 405/AlexaFluor 647 and the number of localizations was calculated from origami images containing single, double and triple clusters corresponding to single dynein (2 GFPs), double dyneins (4 GFPs) and triple dyneins (6 GFPs) (median values, standard deviations and number of clusters analyzed are reported in 2nd row). The sample size chosen was sufficient to ensure (for $\alpha$=0.05) a statistical power value of 1 and the total number of clusters analysed was N=3077, N=1153, N=250 for single, double and triple motors, respectively.

The inventors next purified a modified dimeric *Saccharomyces cerevisiae* dynein motor (Reck-Peterson, S. L. *et al., 2006*) (Methods and Figure 1) whose individual protomers contained both the SNAP-tag and GFP in the motor's tail cargo-binding domain (Derr, N.D. *et al.,* 2012). Because dynein is a homodimer containing two such protomers, each motor thus has two copies of GFP for imaging. The inventors used the SNAP-tag to covalently link the dynein to anti-handle DNA sequences complimentary to the handles at the same 3 positions of the chassis (Methods and Figure 2a). The inventors then immunostained the GFP using a combination of primary anti-GFP antibodies and AlexaFluor405-AlexaFluor647 labeled secondary antibodies (Methods) and then performed super-resolution microscopy (Figure 2g left) and clustering analysis (Figure 2g right). Once again, only those clusters that co-localized with the TAMRA signal were selected and additionally filtered to retain only the clusters that were separated by the expected distance between the handles (see Methods). The counted number of single, double and triple clusters fit well to a binomial distribution with a labeling efficiency of 38% (Figure 6c). This efficiency was only slightly lower than the labeling efficiency for attaching single fluorophores (48%, Figure 6a) suggesting that the main limitation in labeling is due to the attachment of the dynein to the DNA origami, rather than the antibody labeling efficiency. DNA origami immobilized on a Biotin-Streptavidin functionalized glass substrate and on top of cells displayed similar distributions of the number of localizations per cluster (Figure 9). A calibration curve corresponding to the median number of localizations obtained for 1, 2 and 3 clusters (corresponding to 2, 4 and 6 GFPs) was built (Figure 2h and Table 1). The calibration curve obtained in this regime (up to 6 copies of GFP) was roughly linear, suggesting that the antibody labeling efficiency was high and the binding of primary antibody to its target GFP did not reach saturation levels. This calibration curve can be used to extract average protein copy-numbers in a given image by comparing the median number of localizations obtained in the cellular context to the curve. The main advantage of using the DNA origami calibration as opposed to single fluorophores or sparse spots on the sample is the fact that it simultaneously accounts for the stochasticity of the antibody labeling by the fluorophores as well as the binding of the primary and secondary antibodies. Given that most primary and secondary antibodies are polyclonal, this method provides a more precise calibration that properly accounts for the labeling stoichiometry.

To determine whether this method could be used not only to extract average protein copy-numbers but also the percentage of each oligomeric state, the inventors further explored whether the inventors could fit the distribution of the number of localizations per cluster to a functional form. Indeed, the distribution of localizations for single, double or triple clusters (corresponding to 2, 4 and 6 copies of GFP, respectively) could be simultaneously fit using only 2 free parameters ($\mu_1$ and $\mu_2$) assuming that they correspond to the convolutions of respectively 2, 4 and 6 functions f1, where f1 is a log-normal distribution describing the probability distribution of number of localizations obtained by labeling monomeric GFP with A647-conjugated antibodies (see Methods, Figure 2i) (Schmidt, T. et al., 1996 and Moertelmaier, M.,et al.,2005). Therefore, for a general distribution of localizations containing an unknown mixture of oligomeric states, it should be possible to extract both the oligomeric state and the percentage of oligomers corresponding to that particular state by fitting the data to a linear combination of calibration distributions fn obtained as recursively convoluting f1 n-times as has been shown before for single fluorophores Bakker, G. J. *et al* 2012, Torreno-Pina, J. A. *et al* 2014, Schmidt, T. et al., 1996, Moertelmaier, M.,et al.,2005). The inventors validated this idea in multiple ways. First, the inventors generated a synthetic distribution of localizations by combining a known fraction of single, double and triple clusters from the DNA-origami images, which the inventors then fit to a linear combination of log-normal functions fn (Figure 3a). The fitting was performed at varying the number of functions fn. The optimal number of functions (Nmax) was automatically chosen as the one minimizing the fit objective function (Methods). The extracted fraction of single, double and triple motors (2, 4 and 6 GFPs) was in excellent agreement with the expected fractions given sufficient statistics (around 180 datapoints) (Figure 3b, Figure 7a) and the fit provided the best result for Nmax=3 functions (Figure 3c). Second, to test the range of stoichiometries that can be probed with this method, the inventors combined DNA-origami images to generate synthetic distributions of localizations comprising an equal fraction of 1, 4, 8 and 16 motors (2, 8, 16 and 32 GFPs) (Figure 3d). The peaks in the stoichiometry distribution obtained from the fit were in agreement with the chosen oligomeric states (Figure 3d) providing a good correlation with the theoretical data (Figure 7b) and the objective function was minimized at Nmax=20 functions (Figure 3f). Fitting the data to a convolution of more than 20 functions did not change the results (Figure 7c). The amount of statistics needed for accurate fitting increased with increasing stoichiometry (Figure 7a and b). Given sufficient statistics, the method performed well at a large range of stoichiometries, albeit with decreasing performance especially at stoichiometries larger than 16 ($0.99<R<0.87$ for stoichiometries $3<Nmotors<16$ and $0.76<R<0.5$ for stoichiometries $16<Nmotors<32$, Figure 7d). Finally, the inventors attached dynein to 5 handles on the chassis (thus giving rise to up to 5 dimers and 10 copies of GFP). In this case, due to the short distance between the handle positions (28 nm), the inventors could no longer distinguish clusters corresponding to individual dynein motors (Figure 3g). The inventors thus combined together all the localizations coming from each DNA origami structure (identified by the presence of TAMRA signal) and plotted the distribution of localizations (Figure 3h). The fit to a linear combination of calibration functions fn corresponding to one to five GFP dimers (n=2,4,...,10) revealed a combination of 37% single, 44% two, 14% three, 4% four, 1% five dynein motors, fitting well to a binomial distribution for a labeling efficiency of 33%, and in close agreement with the 38% labeling efficiency obtained for triple handles (Figure 3i). The objective function of the fit was minimized at 5 GFP dimers and did not improve by fitting to a larger number of GFPs (Figure 3l).

The inventors finally applied this calibration method to determine copy-numbers of protein complexes imaged in cells. As a first test of a biological structure, we performed immunofluorescence of the nuclear pore complex (NPC) subunit Nup133 fused to GFP, expressed in U2OS cells in the presence of siRNA to knock down the endogenous copy of Nup133 (Figure 4). We chose Nup133 as its stoichiometry has been previously characterized using various methods (Qiu, W. *et al.* 2012) (Figure 4a). Super-resolution images showed ring-like structures as expected (Figure 4b, c), albeit with lower than the 8-fold symmetry per NPC. This is likely due to the incomplete siRNA knockdown and potential limitations with antibody access when the protein copy number is much higher than the linear regime the inventors demonstrated for the DNA-origami. To test a scenario in which the expected range of stoichiometries is more narrow and controlled, the inventors manually sorted the NUP133 images taking into account the number of Nup133 clusters that were visible by eye. The inventors could reliably sort up to 5 Nup133 clusters, since the images of individual clusters started significantly merging together within the resolution limit of STORM for higher order structures. The inventors note that the manual sorting is still prone to some errors as multiple clusters in close proximity may be counted as a single cluster. The inventors then extracted the distribution for the number of localizations per NPC ring containing 1-5 Nup133 clusters (Figure 4d-h), which showed the expected range of stoichiometries considering 4 copies of GFP per Nup133 cluster (Figure 4d-h and Figure 10 a-e). Indeed, for single clusters the stoichiometry ranged from 1 to 4, for double clusters from 2-8 and so on. In addition, the inventors performed cluster analysis on the NPC images without manual sorting to combine clusters belonging to the same NPC ring into one cluster (Figure 4c) and obtained the corresponding distribution of localizations per NPC ring. After the fitting, the inventors obtained a broad distribution of stoichiometries with a maximum stoichiometry of around 30 and a mean stoichiometry of 12 (Figure 4i, black bars and Figure 10f). The maximum stoichiometry is consistent with the expected stoichiometry of 32. Given that the majority of the NPCs in the super-resolution images contained less than the 8 Nup133 clusters (Figure 4i, inset), the inventors expected to obtain an average stoichiometry lower than 32. In the super-resolution images, the most predominantly observed NPC rings contained around 3 NUP133 clusters (Figure 4i, inset), which is consistent with the mean stoichiometry of 12 retrieved from the fit. In addition, weighing the stoichiometries obtained from the sorted data (1-5 rings) (Figure 4d-h) with their occurrence in the super-resolution images (Figure 4i, inset) gave a distribution that matched remarkably well to the experimentally obtained distribution (Figure 4i, red line). Varying the Nmax around the value for which the objective function was minimized did not change the results (Figure 8), while choosing a much smaller Nmax gave rise to isolated peaks at the tail of the stoichiometry distribution (Figure 8). For consistency, the inventors recommend choosing the Nmax for which the objective function is minimum, however the shape of the stoichiometry distribution obtained after the fit can also help guide the choice for Nmax as its tail should show a smooth decay. Finally, similar results were obtained for another subunit of the NPC, NUP107, belonging to the same sub-complex as NUP133 (Figure 5).

In conclusion, the inventors developed a versatile calibration standard that can be used to quantify protein copy number from super-resolution images obtained after fluorescence labeling. Interestingly, the calibration curve obtained for GFP antibody labeling was mostly linear for a range of up to 10 GFPs suggesting that the antibody labeling is efficient and not affected by crowding and steric hindrance. The use of GFP antibodies provides a particularly versatile strategy for quantifying a large number of proteins of interest using the calibration curve reported here. In order to do so, it is important to point out that same imaging and image analysis conditions should be used as detailed in the Methods and Protocol. The inventors used standard imaging buffers, laser powers and acquisition settings that are typical for STORM experiments. Finally, the method the inventors developed is not limited to GFP antibodies and is also applicable to the use of antibodies against any endogenous protein of interest. In addition, it can be used to calibrate nanobody labeling, Halo or SNAP-tag fusions and photoactivatable and photoswitchable fluorescent proteins.

REFERENCES

Oddone, A., Vilanova, I. V., Tam, J. & Lakadamyali, M. Super-resolution imaging with stochastic single-molecule localization: concepts, technical developments, and biological applications. Microscopy research and technique 77, 502-509, doi:10.1002/jemt.22346 (2014).

Durisic, N., Cuervo, L. L. & Lakadamyali, M. Quantitative super-resolution microscopy: pitfalls and strategies for image analysis. Current opinion in chemical biology 20, 22-28, doi:10.1016/j.cbpa.2014.04.005 (2014).

Deschout, H., Shivanandan, A., Annibale, P., Scarselli, M. & Radenovic, A. Progress in quantitative single-molecule localization microscopy. Histochemistry and cell biology 142, 5-17, doi:10.1007/s00418-014-1217-y (2014).

Fricke, F., Beaudouin, J., Eils, R. & Heilemann, M. One, two or three? Probing the stoichiometry of membrane proteins by single-molecule localization microscopy. Sci Rep 5, 14072, doi:10.1038/srep14072 (2015).

Hummer, G., Fricke, F. & Heilemann, M. Model-independent counting of molecules in single-molecule localization microscopy. Mol Biol Cell 27, 3637-3644, doi:10.1091/mbc.E16-07-0525 (2016).

Jungmann, R. et al. Quantitative super-resolution imaging with qPAINT. Nat Methods 13, 439-442, doi:10.1038/nmeth.3804 (2016).

Puchner, E. M., Walter, J. M., Kasper, R., Huang, B. & Lim, W. A. Counting molecules in single organelles with superresolution microscopy allows tracking of the endosome maturation trajectory. Proc Natl Acad Sci U S A 110, 16015-16020, doi:10.1073/pnas. 1309676110 (2013).

Rollins, G. C., Shin, J. Y., Bustamante, C. & Presse, S. Stochastic approach to the molecular counting problem in superresolution microscopy. Proc Natl

*Acad Sci U S A* 112, E110-118, doi:10.1073/pnas.1408071112 (2015). Ricci, M. A., Manzo, C., Garcia-Parajo, M. F., Lakadamyali, M. & Cosma, M. P. Chromatin fibers are formed by heterogeneous groups of nucleosomes invivo. Cell 160, 1145-1158, doi:10.1016/j.cell.2015.01.054 (2015).

Ehmann, N. et al. Quantitative super-resolution imaging of Bruchpilot distinguishes active zone states. Nature communications 5, 4650, doi:10.1038/ncomms5650 (2014).

Bakker, G. J. et al. Lateral mobility of individual integrin nanoclusters orchestrates the onset for leukocyte adhesion. Proc Natl Acad Sci U S A 109, 4869-4874, doi:10.1073/pnas.1116425109 (2012).

Torreno-Pina, J. A. et al. Enhanced receptor-clathrin interactions induced by N-glycan-mediated membrane micropatterning. Proc Natl Acad Sci U S A 111, 11037-11042, doi:10.1073/pnas. 1402041111 (2014).

Derr, N. D. et al. Tug-of-war in motor protein ensembles revealed with a programmable DNA origami scaffold. Science 338, 662-665, doi:10.1126/science.1226734 (2012).

Reck-Peterson, S. L. et al. Single-molecule analysis of dynein processivity and stepping behavior. Cell 126, 335-348, doi:10.1016/j.ce!!.2006.05.046 (2006).

Schmidt, T., Schutz, G. J., Gruber, H. J. & Schindler, H. Local Stoichiometries Determined by Counting Individual Molecules. Anal. Chem. 68, 4397-4401 (1996).

Moertelmaier, M., Brameshuber, M., Linimeier, M., Schutz, G. J. & Stockinger, H. Thinning out clusters while conserving stoichiometry of labeling. Applied Physics Letters 87 (2005).

Lin, C., Perrault, S. D., Kwak, M., Graf, F. & Shih, W. M. Purification of DNA-origami nanostructures by rate-zonal centrifugation. Nucleic acids research 41, e40, doi:10.1093/nar/gks1070 (2013).

Qiu, W. et al. Dynein achieves processive motion using both stochastic and coordinated stepping. Nature structural & molecular biology 19, 193-200, doi:10.1038/nsmb.2205 (2012).

Szymborska, A. et al. Nuclear pore scaffold structure analyzed by super-resolution microscopy and particle averaging. Science 341, 655-658, doi:10.1126/science.1240672 (2013).

**Claims**

1. A method for obtaining a calibration curve for quantifying protein copy number in fluorescence-based super resolution microscopy which comprises

   a) incubating a DNA origami immobilized on a support, wherein the DNA origami comprises handle oligonucleotides protruding from said DNA origami, said handle oligonucleotides being attached to the DNA origami at predetermined positions and at least one tag, with a protein of interest functionalized with oligonucleotides complementary to the handles protruding from said DNA origami, in conditions allowing the hybridization between the oligonucleotides attached to the DNA origami and the oligonucleotides attached to the protein of interest,
   b) recording a super resolution image of the protein of interest which colocalizes with the tag of the DNA origami,
   c) clustering the image obtained in step b) and identifying the clusters separated by the distance between the handles to obtain the number clusters in said image obtained in step b),
   d) fitting a generic probability distribution function depending on a set of parameters $\mu$ to the distribution of the number of localizations $x$ for one predetermined cluster,

$$f_1(\mu;\ x)$$

   and extending it iteratively to larger clusters by using the equation for n= 2, 3... Nmax

$$f_n = f_{n-1} \otimes f_1$$

   where $\otimes$ represent the convolution in respect to the x variables, between two functions and $n_{max}$ is a predetermined maximum number of clusters, and
   e) obtaining a calibration curve by the parameters determined through the fitting procedure described in d), wherein steps d) and e) are executed by a computer,
   and wherein the super resolution image of the protein of interest is obtained by detecting said protein with a fluorescent antibody, fluorescent nanobody or fluorescent halo/snap tag specific for the protein of interest.

2. The method according to claim 1, wherein $f_1$ is

$$f_1(x) = \frac{1}{x\mu_2\sqrt{2\pi}} e^{-\frac{(\ln x - \mu_1)^2}{2\mu_2^2}}$$

3. Method for quantifying protein copy number in a sample imaged with super resolution microscopy which comprises, obtaining a statistical parameter of the number of localizations in a sample having the protein of interest and comparing it with the calibration curve obtained for said protein of interest according to the method of any of claims 1 or 2.

4. Method for determining the percentage of oligomeric state of a protein in a sample imaged with super-resolution microscopy which comprises fitting the overall distribution of the number of localizations obtained in the sample to

$$g(x) = \sum_{n=1}^{N_{max}} \alpha_n f_n(\mu;x)$$

   where $\alpha_n$ represents the weight of the distribution of $n$-mers being $\sum_{n=1}^{N_{max}} \alpha_n = 1$ and $f_n$ is a linear combination of calibration distributions obtained as convoluting function $f_1$, n-times according to:

$$f_n = f_{n-1} \otimes f_1$$

obtained for said protein of interest according to the method of obtaining a calibration curve of any of claims 1 or 2, wherein fittings are performed by optimization of an objective function.

5. The method according to any of claims 1 to 4, wherein the DNA origami comprises 12 parallel DNA double helices, and/or the DNA origami comprises one tag at position 14 of each of the outer helices.

6. The method according to any of claims 1 to 5, wherein the protein of interest is functionalized with oligonucleotides complementary to the handles protruding at any position 0 to 14, particularly at positions 1, 7 and 13 of helix 0.

7. The method according to any of claims 1 to 6, wherein the clusters analyzed in step c) are separated by a distance shorter than 200 nm, such as between $85\pm7$nm and $157\pm17$ nm.

8. The method according to any of claims 1 to 7 wherein the super resolution image is obtained by STORM.

9. A computer readable storage medium comprising a program stored thereon and instructions which, when the program is executed by a computer, cause the computer to carry out step d)-e) of the method according to any of claims 1 to 8.

10. A kit comprising

a) a DNA origami attachable to a support comprising handle sequences protruding from said DNA origami and at least one tag, optionally the DNA origami is protected from degradation,
b) reagents suitable for obtaining a super resolution image of a protein of interest wherein the reagents for obtaining a super resolution image of a protein of interest comprise

i) an antibody or nanobody specific for the protein of interest having at least one fluorophore, or
ii) a primary antibody specific for the protein of interest and a secondary antibody having at least one fluorophore,
and

c) a computer-readable medium comprising instructions which, when executed by a computer, cause the computer to carry out step d)-e) of the method according to any of claims 1 to 8,

11. The kit according to claim 10 wherein the DNA origami is attached to a support.

12. The kit according to any of claims 10 or 11, wherein the DNA origami comprises 12 parallel DNA double helices and/or one tag at position 14 of each of the outer helices.

13. Use of a kit according to any of claims 10 to 12 for obtaining a calibration curve for quantifying protein copy number in immunofluorescence-based super resolution microscopy, for quantifying protein copy number in a sample imaged with super resolution microscopy and for determining the percentage of oligomeric state of a protein in a sample imaged with super-resolution microscopy.

**Patentansprüche**

1. Verfahren zur Gewinnung einer Kalibrierungskurve zur Quantifizierung der Proteinkopienzahl in der Fluoreszenz-basierten Superauflösungsmikroskopie, welches umfasst:

a) Inkubieren eines auf einem Träger immobilisierten DNA-Origamis, wobei das DNA-Origami Griff-Oligonukleotide umfasst, die aus dem DNA-Origami herausragen, wobei die Griff-Oligonukleotide an vorbestimmten Positionen an das DNA-Origami gebunden sind, und mindestens eine Markierung, mit einem Protein von Interesse, das mit Oligonukleotiden funktionalisiert ist, die komplementär zu den aus dem DNA-Origami herausragenden Griffen sind, unter Bedingungen, die die Hybridisierung zwischen den an das DNA-Origami gebundenen Oligonukleotiden und den an das Protein von Interesse gebundenen Oligonukleotiden ermöglichen,
b) Aufnehmen eines superaufgelösten Bildes von dem Protein von Interesse, das mit der Markierung des DNA-Origamis kolokalisiert,
c) Clustering des in Schritt b) erhaltenen Bildes und Identifizieren der durch den Abstand zwischen den Griffen getrennten Cluster, um die Anzahl der Cluster in dem in Schritt b) erhaltenen Bild zu erhalten,

d) Anpassen einer generischen Wahrscheinlichkeitsverteilungsfunktion, die von einem Satz von Parametern $\mu$ abhängt, an die Verteilung der Anzahl von Lokalisierungen $x$ für ein vorbestimmtes Cluster,

$$f_1(\mu; x)$$

und iterative Erweiterung auf größere Cluster, unter Verwendung der Gleichung für n= 2, 3 ... Nmax

$$f_n = f_{n-1} \otimes f_1$$

wobei $\otimes$ die Faltung in Bezug auf die x Variablen zwischen zwei Funktionen darstellt und $n_{max}$ eine vorgegebene maximale Anzahl von Clustern ist, und

e) Gewinnen einer Kalibrierungskurve durch die mittels des in d) beschriebenen Anpassungsverfahren bestimmten Parameter, wobei die Schritte d) und e) von einem Computer ausgeführt werden

und wobei das superaufgelöste Bild des Proteins von Interesse durch Detektion des Proteins mit einem fluoreszierenden Antikörper, fluoreszierenden Nanokörper oder fluoreszierenden Halo-/Snap-Tag, die spezifisch für das Protein von Interesse sind, erhalten wird.

2. Verfahren nach Anspruch 1, wobei $f_1$ ist:

$$f_1(x) = \frac{1}{x\mu_2\sqrt{2\pi}} e^{-\frac{(\ln x - \mu_1)^2}{2\mu_2^2}}$$

3. Verfahren zur Quantifizierung der Proteinkopienzahl in einer Probe, die mit Superauflösungsmikroskopie abgebildet wurde, umfassend das Erhalten eines statistischen Parameters der Anzahl von Lokalisierungen in einer Probe mit dem Protein von Interesse, und das Vergleichen dieses Parameters mit der Kalibrierungskurve, die für das Protein von Interesse gemäß dem Verfahren nach einem der Ansprüche 1 und 2 erhalten wurde.

4. Verfahren zur Bestimmung des prozentualen Anteils des oligomeren Zustands eines Proteins in einer Probe, die mit Superauflösungsmikroskopie abgebildet wurde, welches die Anpassung der Gesamtverteilung der Anzahl der in der Probe erhaltenen Lokalisationen an

$$g(x) = \sum_{n=1}^{N_{max}} \alpha_n f_n(\mu; x)$$

umfasst, wobei $\alpha_n$ die Gewichtung der Verteilung von n-Meren darstellt, mit $\sum_{n=1}^{N_{max}} \alpha_n = 1$ und
$f_n$ eine lineare Kombination von Kalibrierungsverteilungen ist, die als Faltungsfunktion $f_1$ erhalten wird, n-mal gemäß:

$$f_n = f_{n-1} \otimes f_1$$

die für das Protein von Interesse gemäß dem Verfahren zur Gewinnung einer Kalibrierungskurve nach einem der Ansprüche 1 oder 2 erhalten wird, wobei die Anpassungen durch Optimierung einer Zielfunktion durchgeführt werden.

5. Verfahren nach einem der Ansprüche 1 bis 4, wobei das DNA-Origami 12 parallele DNA-Doppelhelices umfasst und/oder das DNA-Origami ein Tag an Position 14 jeder der äußeren Helices umfasst.

6. Verfahren nach einem der Ansprüche 1 bis 5, wobei das Protein von Interesse mit Oligonukleotiden funktionalisiert wird, die komplementär zu den Griffen sind, die an einer beliebigen Position 0 bis 14 herausragen, insbesondere an den Positionen 1, 7 und 13 der Helix 0.

7. Verfahren nach einem der Ansprüche 1 bis 6, wobei die in Schritt c) analysierten Cluster durch einen Abstand von weniger als 200 nm getrennt sind, beispielsweise zwischen $85\pm7$ nm und $157\pm17$ nm.

8. Verfahren nach einem der Ansprüche 1 bis 7, wobei das superaufgelöste Bild durch STORM erhalten wird.

9. Computerlesbares Speichermedium mit einem darauf gespeicherten Programm und Instruktionen, die, wenn das Programm von einem Computer ausgeführt wird, den Computer veranlassen, die Schritte d)-e) des Verfahrens nach einem der Ansprüche 1 bis 8 auszuführen.

10. Kit, umfassend

   a) ein DNA-Origami, das an einen Träger gebunden werden kann, welches Griff-Sequenzen, die aus dem DNA-Origami herausragen, und mindestens ein Tag umfasst, wobei das DNA-Origami gegebenenfalls vor Abbau geschützt ist,
   b) Reagenzien, die geeignet sind, um ein superaufgelöstes Bild eines Proteins von Interesse zu erhalten, wobei die Reagenzien zum Erhalt eines superaufgelösten Bildes eines Proteins von Interesse Folgendes umfassen:

      i) einen Antikörper oder Nanokörper, der für das Protein von Interesse spezifisch ist und mindestens einen Fluorophor aufweist, oder
      ii) einen primären Antikörper, der für das Protein von Interesse spezifisch ist, und einen sekundären Antikörper mit mindestens einem Fluorophor, und

   c) ein computerlesbares Medium, das Instruktionen enthält, die, wenn sie von einem Computer ausgeführt werden, den Computer veranlassen, Schritt d)-e) des Verfahrens nach einem der Ansprüche 1 bis 8 auszuführen.

11. Kit nach Anspruch 10, wobei das DNA-Origami an einen Träger gebunden ist.

12. Kit nach Anspruch 10 oder 11, wobei das DNA-Origami 12 parallele DNA-Doppelhelices und/oder ein Tag an Position 14 jeder der äußeren Helices umfasst.

13. Verwendung eines Kits nach einem der Ansprüche 10 bis 12 zur Gewinnung einer Kalibrierungskurve zur Quantifizierung der Proteinkopienzahl in Immunfluoreszenzbasierten Superauflösungsmikroskopie, zur Quantifizierung der Proteinkopienzahl in einer mit Superauflösungsmikroskopie abgebildeten Probe und zur Bestimmung des prozentualen Anteils des oligomeren Zustands eines Proteins in einer mit Superauflösungsmikroskopie abgebildeten Probe.

**Revendications**

1. Procédé d'obtention d'une courbe d'étalonnage pour quantifier un nombre de copies de protéine par microscopie à super résolution basée sur la fluorescence, qui comprend

   a) l'incubation d'un origami d'ADN immobilisé sur un support, dans lequel l'origami d'ADN comprend des oligonucléotides poignées faisant saillie à partir dudit origami d'ADN, lesdits oligonucléotides poignées étant fixés à l'origami d'ADN aux positions prédéterminées et au moins une étiquette, avec une protéine d'intérêt fonctionnalisée avec des oligonucléotides complémentaires aux poignées faisant saillie à partir dudit origami d'ADN, dans des conditions permettant l'hybridation entre les oligonucléotides fixés à l'origami d'ADN et les oligonucléotides fixés à la protéine d'intérêt,
   b) enregistrer une image à super résolution de la protéine d'intérêt colocalisée avec l'étiquette de l'origami d'ADN,
   c) regrouper l'image obtenue à l'étape b) et identifier les groupes séparés par la distance entre les poignées pour obtenir le nombre de groupes dans ladite image obtenue à l'étape b),
   d) ajuster une fonction de distribution de probabilité générique dépendant d'un ensemble de paramètres $\mu$ à la distribution du nombre de localisations x pour un groupe prédéterminé,

$$f_1\left(\mu;x\right)$$

et l'étendre de manière itérative à des groupes plus grands en utilisant l'équation pour n= 2, 3... Nmax

$$f_n = f_{n-1} \otimes f_1$$

où $\otimes$ représente la convolution par rapport aux variables x, entre deux fonctions et $n_{max}$ est un nombre maximal prédéterminé de groupes, et

e) l'obtention d'une courbe d'étalonnage avec les paramètres déterminés par la procédure d'ajustement décrite en d), où les étapes d) et e) sont réalisées par un ordinateur,

et où l'image à super résolution de la protéine d'intérêt est obtenue en détectant ladite protéine avec un anticorps fluorescent, un nanocorps fluorescent ou une étiquette halo/snap fluorescente spécifique à la protéine d'intérêt.

2. Procédé selon la revendication 1, dans lequel $f_1$ est

$$f_1(x) = \frac{1}{x\mu_2\sqrt{2\pi}} e^{-\frac{(\ln x - \mu_1)^2}{2\mu_2^2}}$$

3. Procédé pour quantifier un nombre de copies de protéine dans un échantillon imagé par microscopie à super résolution qui comprend l'obtention d'un paramètre statistique du nombre de localisations dans un échantillon ayant la protéine d'intérêt et sa comparaison avec la courbe d'étalonnage obtenue pour ladite protéine d'intérêt selon le procédé de l'une quelconque des revendications 1 ou 2.

4. Procédé pour déterminer le pourcentage d'état oligomérique d'une protéine dans un échantillon imagé par microscopie à super résolution qui comprend l'ajustement de la distribution globale du nombre de localisations obtenues dans l'échantillon à

$$g(x) = \sum_{n=1}^{N_{max}} \alpha_n f_n(\mu; x)$$

où $\alpha_n$ représente le poids de la distribution des n-mères avec $\sum_{n=1}^{N_{max}} \alpha_n = 1$ et $f_n$ est une combinaison linéaire de distributions de calibration obtenues en tant que fonction de convolution, n-fois selon :

$$f_n = f_{n-1} \otimes f_1$$

obtenue pour ladite protéine d'intérêt selon le procédé d'obtention d'une courbe d'étalonnage selon l'une quelconque des revendications 1 ou 2, dans lequel des ajustements sont réalisés par optimisation d'une fonction objectif.

5. Procédé selon l'une quelconque des revendications 1 à 4, dans lequel l'origami d'ADN comprend 12 doubles hélices d'ADN parallèles, et/ou l'origami d'ADN comprend une étiquette en position 14 de chacune des hélices externes.

6. Procédé selon l'une quelconque des revendications 1 à 5, dans lequel la protéine d'intérêt est fonctionnalisée avec des oligonucléotides complémentaires aux poignées faisant saillie à une position quelconque de 0 à 14, en particulier aux positions 1, 7 et 13 de l'hélice 0.

7. Procédé selon l'une quelconque des revendications 1 à 6, dans lequel les groupes analysés à l'étape c) sont séparés par une différence plus petite que 200 nm, tel qu'entre $85 \pm 7$ nm et $157 \pm 17$ nm.

8. Procédé selon l'une quelconque des revendications 1 à 7 dans lequel l'image à super résolution est obtenue par STORM.

9. Support de stockage lisible par un ordinateur comprenant un programme stocké sur celui-ci et des instructions qui, lorsque le programme est exécuté par un ordinateur, amènent l'ordinateur à réaliser l'étape d)-e) du procédé selon l'une quelconque des revendications 1 à 8.

10. Kit comprenant

a) un origami d'ADN pouvant être fixé à un support comprenant des séquences poignées faisant saillie à partir dudit origami d'ADN et au moins une étiquette, optionnellement l'origami d'ADN est protégé d'une dégradation,
b) des réactifs adaptés pour obtenir une image à super résolution d'une protéine d'intérêt dans lequel les réactifs pour obtenir une image à super résolution d'une protéine d'intérêt comprennent

i) un anticorps ou nanocorps spécifique à la protéine d'intérêt ayant au moins un fluorophore, ou
ii) un anticorps primaire spécifique à la protéine d'intérêt et un anticorps secondaire ayant au moins un fluorophore,
et

c) un support lisible par ordinateur comprenant des instructions qui, lorsqu'elles sont exécutées par un ordinateur, amènent l'ordinateur à réaliser l'étape d)-e) du procédé selon l'une quelconque des revendications 1 à 8.

11. Kit selon la revendication 10 dans lequel l'origami d'ADN est fixée à un support.

12. Kit selon l'une quelconque des revendications 10 ou 11, dans lequel l'origami d'ADN comprend 12 doubles hélices d'ADN parallèles et/ou une étiquette en position 14 de chacune des hélices externes.

13. Utilisation d'un kit selon l'une quelconque des revendications 10 à 12 pour l'obtention d'une courbe d'étalonnage pour quantifier un nombre de copies de protéine en microscopie à super résolution basée sur l'immunofluorescence, pour quantifier un nombre de copies de protéine dans un échantillon imagé par microscopie à super résolution et pour déterminer le pourcentage d'état oligomérique d'une protéine dans un échantillon imagé par microscopie à super résolution.

Fig. 1

Fig. 2

Fig. 2 (Cont.)

Fig. 3

Fig. 3 (Cont.)

Fig. 4

Fig. 4 (Cont.)

Fig. 4 (Cont.)

Fig. 5

Fig. 5 (Cont.)

Fig. 6

Fig. 7

Fig. 8

Fig. 8 (Cont.)

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013261019 A
- US 2014057805 A
- WO 2013090360 A
- WO 2009085218 A
- EP 2378343 A
- WO 2008091296 A

### Non-patent literature cited in the description

- **ZANACCHI et al.** *Biophysical Journal,* 2017, vol. 112 (3
- **JUNGMANN et al.** *Nature Methods,* 2014, vol. 11, 313-318
- **DAI et al.** *Nature Nanotechnology,* 2016, vol. 11, 798-807
- **DERR et al.** *Science,* 2012, vol. 338, 662-665
- **GOODMAN, B. S. et al.** *Meth. Enzymol.,* 2014, vol. 540, 169-188
- Overview of principles of hybridization and the strategy of nucleic acid assays. **TIJSSEN S.** Laboratory Techniques in Biochemistry and Molecular Biology. Elsevier Science Publishers, 1993
- **DOWDY ; WEARDEN.** Statistics for Research. John Wiley & Sons, 1983
- **ODDONE, A. ; VILANOVA, I. V. ; TAM, J. ; LAKADAMYALI, M.** Super-resolution imaging with stochastic single-molecule localization: concepts, technical developments, and biological applications. *Microscopy research and technique,* 2014, vol. 77, 502-509
- **DURISIC, N. ; CUERVO, L. L. ; LAKADAMYALI, M.** Quantitative super-resolution microscopy: pitfalls and strategies for image analysis. *Current opinion in chemical biology,* 2014, vol. 20, 22-28
- **DESCHOUT, H. ; SHIVANANDAN, A. ; ANNIBALE, P. ; SCARSELLI, M. ; RADENOVIC, A.** Progress in quantitative single-molecule localization microscopy. *Histochemistry and cell biology,* 2014, vol. 142, 5-17
- **FRICKE, F. ; BEAUDOUIN, J. ; EILS, R. ; HEILEMANN, M.** One, two or three? Probing the stoichiometry of membrane proteins by single-molecule localization microscopy. *Sci Rep,* 2015, vol. 5, 14072
- **HUMMER, G. ; FRICKE, F. ; HEILEMANN, M.** Model-independent counting of molecules in single-molecule localization microscopy. *Mol Biol Cell,* 2016, vol. 27, 3637-3644
- **JUNGMANN, R. et al.** Quantitative super-resolution imaging with qPAINT. *Nat Methods,* 2016, vol. 13, 439-442
- **PUCHNER, E. M. ; WALTER, J. M. ; KASPER, R. ; HUANG, B. ; LIM, W. A.** Counting molecules in single organelles with superresolution microscopy allows tracking of the endosome maturation trajectory. *Proc Natl Acad Sci U S A,* 2013, vol. 110, 16015-16020
- **ROLLINS, G. C. ; SHIN, J. Y. ; BUSTAMANTE, C. ; PRESSE, S.** Stochastic approach to the molecular counting problem in superresolution microscopy. *Proc Natl Acad Sci USA,* 2015, vol. 112
- **RICCI, M. A. ; MANZO, C. ; GARCIA-PARAJO, M. F. ; LAKADAMYALI, M. ; COSMA, M. P.** Chromatin fibers are formed by heterogeneous groups of nucleosomes in vivo. *Cell,* 2015, vol. 160, 1145-1158
- **EHMANN, N. et al.** Quantitative super-resolution imaging of Bruchpilot distinguishes active zone states. *Nature communications,* 2014, vol. 5, 4650
- **BAKKER, G. J. et al.** Lateral mobility of individual integrin nanoclusters orchestrates the onset for leukocyte adhesion. *Proc Natl Acad Sci U S A,* 2012, vol. 109, 4869-4874
- **TORRENO-PINA, J. A. et al.** Enhanced receptor-clathrin interactions induced by N-glycan-mediated membrane micropatterning. *Proc Natl Acad Sci U S A,* 2014, vol. 111, 11037-11042
- **DERR, N. D. et al.** Tug-of-war in motor protein ensembles revealed with a programmable DNA origami scaffold. *Science,* 2012, vol. 338, 662-665
- **RECK-PETERSON, S. L. et al.** Single-molecule analysis of dynein processivity and stepping behavior. *Cell,* 2006, vol. 126, 335-348
- **SCHMIDT, T. ; SCHUTZ, G. J. ; GRUBER, H. J. ; SCHINDLER, H.** Local Stoichiometries Determined by Counting Individual Molecules. *Anal. Chem.,* 1996, vol. 68, 4397-4401
- **MOERTELMAIER, M. ; BRAMESHUBER, M. ; LINIMEIER, M. ; SCHUTZ, G. J. ; STOCKINGER, H.** Thinning out clusters while conserving stoichiometry of labeling. *Applied Physics Letters,* 2005, vol. 87

- **LIN, C. ; PERRAULT, S. D. ; KWAK, M. ; GRAF, F. ; SHIH, W. M.** Purification of DNA-origami nanostructures by rate-zonal centrifugation. *Nucleic acids research,* 2013, vol. 41, e40
- **QIU, W. et al.** Dynein achieves processive motion using both stochastic and coordinated stepping. *Nature structural & molecular biology,* 2012, vol. 19, 193-200
- **SZYMBORSKA, A. et al.** Nuclear pore scaffold structure analyzed by super-resolution microscopy and particle averaging. *Science,* 2013, vol. 341, 655-658

SEQUENCE LISTING

<110> FUNDACIO INSTITUT DE CIENCIES FOTONIQUES SMITH COLLEGE FUNDACIO UNIVERSITARIA BALMES

<120> METHOD FOR QUANTIFYING PROTEIN COPY-NUMBER

<130> P14741EP00

<160> 10

<170> PatentIn version 3.5

<210> 1
<211> 44
<212> DNA
<213> Artificial sequence

<220>
<223> HO #1

<400> 1
aactgttggg aacgttccgg caattcacta cttaccactc tacc 44

<210> 2
<211> 44
<212> DNA
<213> Artificial sequence

<220>
<223> HO #3

<400> 2
agatacattt cgattgcctg agattcacta cttaccactc tacc 44

<210> 3
<211> 44
<212> DNA
<213> Artificial sequence

<220>
<223> HO #7

<400> 3
gagggttgat atgctttcga ggtttcacta cttaccactc tacc 44

<210> 4
<211> 44
<212> DNA
<213> Artificial sequence

<220>
<223> HO #11

<400> 4
acaaattctt actaagaacg cgattcacta cttaccactc tacc 44

<210> 5
<211> 44
<212> DNA
<213> Artificial sequence

<220>
<223> HO #13

<400> 5
gtcaatagat aatacctgag caattcacta cttaccactc tacc 44

<210> 6
<211> 29
<212> DNA
<213> Artificial sequence

<220>
<223> H7 #1. Sequence with a 3'biotin tag.

<400> 6
gtaagctttc agaggtggag ccgtttttt 29

<210> 7
<211> 29
<212> DNA
<213> Artificial sequence

<220>
<223> H4 #5. Sequence with a 3'biotin tag

<400> 7
aaggcttgcc ctctcaaatg ctttttttt 29

<210> 8
<211> 29
<212> DNA
<213> Artificial sequence

<220>
<223> H7 #9. Sequence with a 3'biotin tag.

<400> 8
aaaaaattca tatggtttac cagtttttt 29

<210> 9
<211> 29
<212> DNA
<213> Artificial sequence

<220>
<223> H4 #13. Sequence with a 3'biotin tag.

<400> 9
agtaacatta tccaatatat gtgtttttt 29

<210> 10
<211> 21
<212> DNA
<213> Artificial sequence

<220>
<223> Complementary oligonucleotide A*

<400> 10
ggtagagtgg taagtagtga a 21